# EUROPÄISCHE PATENTSCHRIFT

(11) **EP 0 942 652 B1**
(45) Veröffentlichungstag und Bekanntmachung des Hinweises auf die Patenterteilung: **23.02.2005**
(21) Anmeldenummer: 97951988.1
(22) Anmeldetag: 27.11.1997
(51) Int. Cl.: A01N 47/36, C07D 409/12

(54) **SUBSTITUIERTE THIENYL(AMINO)SULFONYL(THIO)HARNSTOFFE ALS HERBIZIDE**
SUBSTITUTED THIENYL(AMINO)SULPHONYL(THIO)UREAS AS HERBICIDES
THIENYL(AMINO)SULFONYL(THIO)UREES SUBSTITUEES S'UTILISANT COMME HERBICIDES

(30) Priorität: 09.12.1996 DE 19651037
(43) Veröffentlichungstag der Anmeldung: 22.09.1999
(73) Patentinhaber: Bayer CropScience AG, 40789 Monheim (DE)
(72) Erfinder: GESING, Ernst, Rudolf, F., D-40699 Erkrath (DE); JANSEN, Johannes, Rudolf, D-40789 Monheim (DE); MÜLLER, Klaus-Helmut, D-40593 Düsseldorf (DE); PHILIPP, Ulrich, D-50674 Köln (DE); DOLLINGER, Markus, D-51381 Leverkusen (DE)
(86) Internationale Anmeldenummer: PCT/EP1997/006617
(87) Internationale Veröffentlichungsnummer: WO 1998/025467

(56) Entgegenhaltungen:
- EP-A- 0 030 140
- EP-A- 0 041 404
- US-A- 4 877 440

## Beschreibung

Die Erfindung betrifft neue substituierte Thienyl(amino)sulfonyl(thio)harnstoffe, Verfahren zu ihrer Herstellung und ihre Verwendung als Herbizide.

Es ist bereits bekannt, daß bestimmte substituierte Thienylsulfonylharnstoffe herbizide Eigenschaften aufweisen (vgl. US 4127405, US 4169719, US 4398939, US 4523943, US 4877440). Die herbizide Wirksamkeit dieser bekannten Verbindungen ist jedoch nicht in allen Belangen zufriedenstellend.

Es wurden nun die neuen substituierten Thienyl(amino)sulfonyl(thio)harnstoffe der allgemeinen Formel (I) 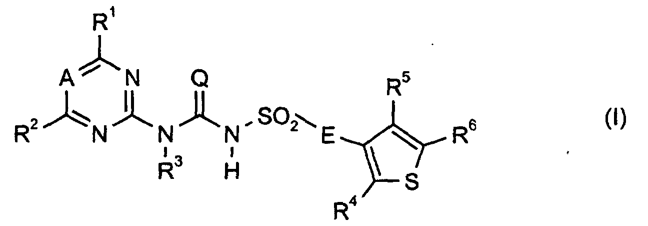 in welcher
- A: für Stickstoff oder eine CH-Gruppierung steht,
- E: für eine Einfachbindung oder eine NH-Gruppierung steht,
- Q: für Sauerstoff oder Schwefel steht,
- R¹: für Wasserstoff, Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, für jeweils gegebenenfalls durch Cyano, Halogen, C₁-C₄-Alkyl oder C₁-C₄-Alkoxy substituiertes Cycloalkyl oder Cycloalkyloxy mit jeweils 3 bis 6 Kohlenstoffatomen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy steht,
- R²: für Wasserstoff oder Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, für jeweils gegebenenfalls durch Cyano, Halogen, C₁-C₄-Alkyl oder C₁-C₄-Alkoxy substituiertes Cycloalkyl oder Cycloalkyloxy mit jeweils 3 bis 6 Kohlenstoffatomen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy steht,
- R³: für Wasserstoff oder gegebenenfalls durch C₁-C₄-Alkoxy, C₁-C₄-Alkyl-carbonyl oder C₁-C₄-Alkoxy-carbonyl substituiertes Alkyl mit 1 bis 4 Kohlenstoffatomen steht,
- R⁴: für C₁-C₄-Alkyl steht,
- R⁵: für C₁-C₄-Alkyl steht und
- R⁶: für Wasserstoff, für Cyano, Halogen, für gegebenenfalls durch Cyano, Halogen oder C₁-C₄-Alkoxy substituiertes C₁-C₄-Alkyl, für jeweils gegebenenfalls durch Cyano oder Halogen substituiertes C₂-C₄-Alkenyl oder C₂-C₄-Alkinyl, für gegebenenfalls durch Cyano, Halogen oder C₁-C₄-Alkoxy substituiertes C₁-C₄-Alkoxy, oder für jeweils gegebenenfalls durch Cyano oder Halogen substituiertes C₂-C₄-Alkenyloxy oder C₂-C₄-Alkinyloxy steht,
sowie Salze von Verbindungen der Formel (I) gefunden.

Man erhält die neuen substituierten Thienyl(amino)sulfonyl(thio)harnstoffe der allgemeinen Formel (I), wenn man
- für den Fall, daß in der allgemeinen Formel (I) E für eine Einfachbindung steht -
(a) Aminoazine der allgemeinen Formel (II) 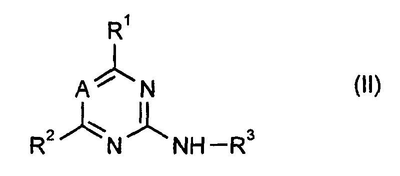 in welcher
   A, R¹, R² und R³ die oben angegebene Bedeutung haben,
   mit Thienylsulfonyliso(thio)cyanaten der allgemeinen Formel (III) 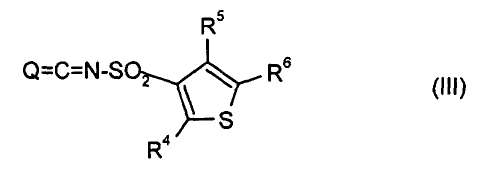 in welcher
   Q, R⁴, R⁵ und R⁶ die oben angegebene Bedeutung haben,
   gegebenenfalls in Gegenwart eines Reaktionshilfsmittels und gegebenenfalls in Gegenwart eines Verdünnungsmittels umsetzt, oder
(b) substituierte Aminoazine der allgemeinen Formel (IV) 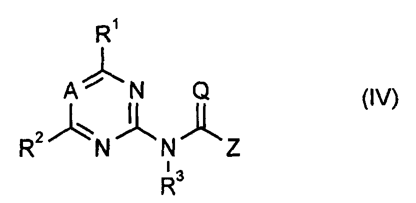 in welcher
   - A, Q, R¹, R² und R³: die oben angegebene Bedeutung haben und
   - Z: für Halogen, Alkoxy oder Aryloxy steht,
   mit Thiophensulfonamiden der allgemeinen Formel (V) 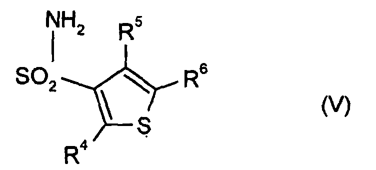 in welcher
   R⁴, R⁵ und R⁶ die oben angegebene Bedeutung haben,
   gegebenenfalls in Gegenwart eines Reaktionshilfsmittels und gegebenenfalls in Gegenwart eines Verdünnungsmittels umsetzt, oder
(c) Aminoazine der allgemeinen Formel (II) 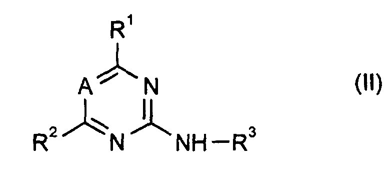 in welcher
   A, R¹, R² und R³ die oben angegebene Bedeutung haben,
   mit substituierten Thiophensulfon(thio)amiden der allgemeinen Formel (VI) 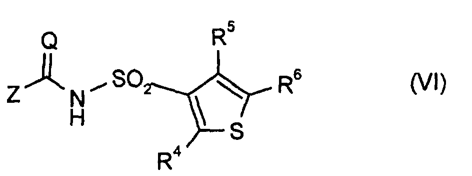 in welcher
   Q, R⁴, R⁵ und R⁶ die oben angegebene Bedeutung haben und
   Z für Halogen, Alkoxy oder Aryloxy steht,
   gegebenenfalls in Gegenwart eines Reaktionshilfsmittels und gegebenenfalls in Gegenwart eines Verdünnungsmittels umsetzt,
   oder wenn man - für den Fall, daß E für eine NH-Gruppierung steht -
(d) Aminoazine der allgemeinen Formel (II) 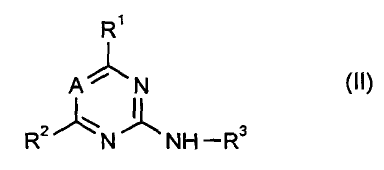 in welcher
   A, R¹, R² und R³ die oben angegebene Bedeutung haben,
   mit Chlorsulfonyliso(thio)cyanat, gegebenenfalls in Gegenwart eines Verdünnungsmittels, umsetzt und die hierbei gebildeten Chlorsulfonylharnstoffe der allgemeinen Formel (VII) 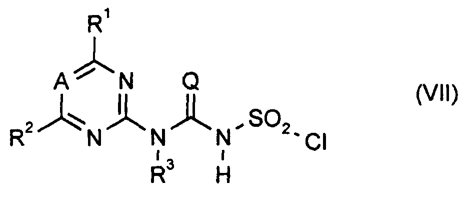 in welcher
   A, Q, R¹, R² und R³ die oben angegebene Bedeutung haben,
   mit Aminothiophenen der allgemeinen Formel (VIII) 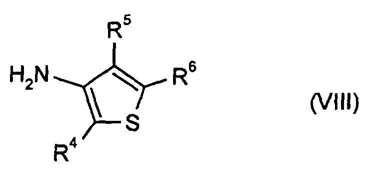 in welcher
   R⁴, R⁵ und R⁶ die oben angegebene Bedeutung haben,
   gegebenenfalls in Gegenwart eines Reaktionshilfsmittels und gegebenenfalls in Gegenwart eines Verdünnungsmittels umsetzt,
   und gegebenenfalls die nach den Verfahren (a), (b), (c) oder (d) erhaltenen Verbindungen der Formel (I) nach üblichen Methoden in Salze überführt.

Die neuen substituierten Thienyl(amino)sulfonyl(thio)harnstoffe der allgemeinen Formel (I) zeichnen sich durch starke herbizide Wirksamkeit aus.

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (I), in welcher A, E, Q, R¹, R², R³, R⁴, R⁵ und R⁶ die oben vorzugsweise angegebene Bedeutung haben.

Gegenstand der Erfindung sind insbesondere Verbindungen der Formel (I), in welcher
- A: für Stickstoff oder eine CH-Gruppierung steht,
- E: für eine Einfachbindung oder eine NH-Gruppierung steht,
- Q: für Sauerstoff oder Schwefel steht,
- R¹: für Wasserstoff, Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
- R²: für Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,

- R³: für Wasserstoff oder gegebenenfalls durch Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl oder Ethyl steht,
- R⁴: für Methyl, Ethyl, n- oder i-Propyl, n-, i- oder s-Butyl steht,
- R⁵: für Methyl, Ethyl, n- oder i-Propyl, n-, i- oder s-Butyl steht und
- R⁶: für Wasserstoff, für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methoxy oder Ethoxy substituiertes Methoxy, Ethoxy, n- oder i-Propoxy, n-, i-, s- oder t-Butoxy, oder für jeweils gegebenenfalls durch Cyano, Fluor oder Chlor substituiertes Propenyloxy, Butenyloxy, Propinyloxy oder Butinyloxy steht.

Eine ganz besonders bevorzugte Gruppe sind diejenigen Verbindungen der Formel (I), in welcher
- A: für Stickstoff oder eine CH-Gruppierung steht,
- E: für eine Einfachbindung steht,
- Q: für Sauerstoff oder Schwefel steht,
- R¹: für Wasserstoff, Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
- R²: für Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
- R³: für Wasserstoff oder gegebenenfalls durch Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl oder Ethyl steht,
- R⁴: für Methyl, Ethyl, n- oder i-Propyl, n-, i- oder s-Butyl steht,
- R⁵: für Methyl, Ethyl, n- oder i-Propyl, n-, i- oder s-Butyl steht und
- R⁶: für Wasserstoff, für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methoxy oder Ethoxy substituiertes Methoxy, Ethoxy, n- oder i-Propoxy, n-, i-, s- oder t-Butoxy, oder für jeweils gegebenenfalls durch Cyano, Fluor oder Chlor substituiertes Propenyloxy, Butenyloxy, Propinyloxy oder Butinyloxy steht.

Eine weitere ganz besonders bevorzugte Gruppe sind diejenigen Verbindungen der Formel (I), in welcher
- A: für Stickstoff oder eine CH-Gruppierung steht,
- E: für eine NH-Gruppierung steht,
- Q: für Sauerstoff oder Schwefel steht,
- R¹: für Wasserstoff, Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
- R²: für Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
- R³: für Wasserstoff oder gegebenenfalls durch Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl oder Ethyl steht,
- R⁴: für Methyl, Ethyl, n- oder i-Propyl, n-, i- oder s-Butyl steht,
- R⁵: für Methyl, Ethyl, n- oder i-Propyl, n-, i- oder s-Butyl steht und
- R⁶: für Wasserstoff, für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methoxy oder Ethoxy substituiertes Methoxy, Ethoxy, n- oder i-Propoxy, n-, i-, s- oder t-Butoxy, oder für jeweils gegebenenfalls durch Cyano, Fluor oder Chlor substituiertes Propenyloxy, Butenyloxy, Propinyloxy oder Butinyloxy steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Verwendet man beispielsweise 2-Amino-4-methoxy-6-methyl-pyrimidin und 2-Ethyl-4-trifluormethyl-thien-3-yl-sulfonylisocyanat als Ausgangsstoffe, so kann der Reaktionsablauf beim erfindungsgemäßen Verfahren (a) durch das folgende Formelschema skizziert werden:

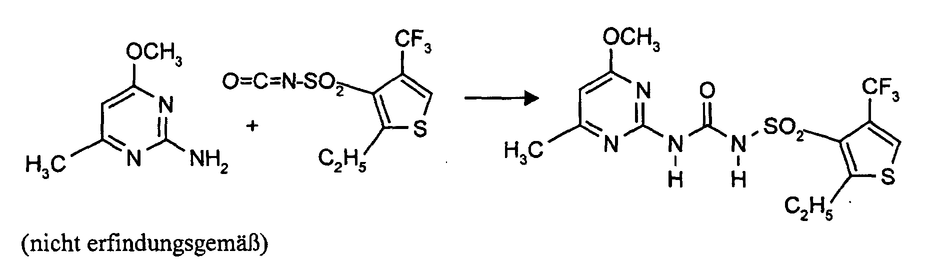

Verwendet man beispielsweise 2-Methoxycarbonylamino-4-methoxy-6-trifluormethyl-1,3,5-triazin und 4-Ethyl-2-methyl-thiophen-3-sulfonamid als Ausgangsstoffe, so kann der Reaktionsablauf beim erfindungsgemäßen Verfahren (b) durch das folgende Formelschema skizziert werden:

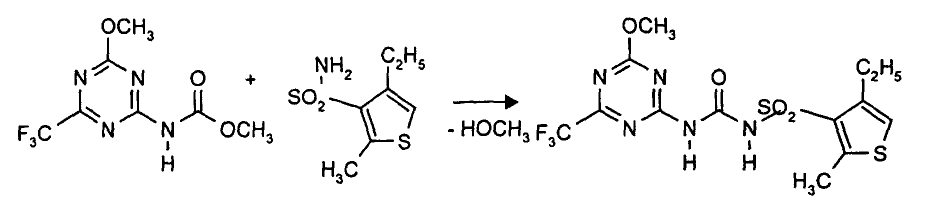

Verwendet man beispielsweise 2-Amino-4-chlor-6-methoxy-pyrimidin und N-(2-Chlor-4-methyl-thien-3-yl-sulfonyl)-O-phenyl-urethan als Ausgangsstoffe, so kann der Reaktionsablauf beim erfindungsgemäßen Verfahren (c) durch das folgende Formelschema skizziert werden:

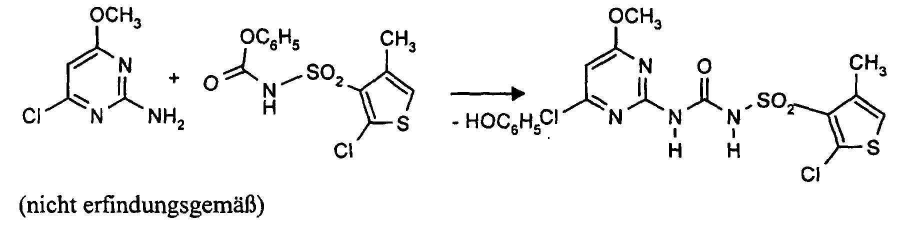

Verwendet man beispielsweise 2-Amino-4-methoxy-6-methyl-1,3,5-triazin und Chlorsulfonylisocyanat sowie anschließend 3-Amino-4-chlor-2-cyano-thiophen als Ausgangsstoffe, so kann der Reaktionsablauf beim erfindungsgemäßen Verfahren (d) durch das folgende Formelschema skizziert werden:

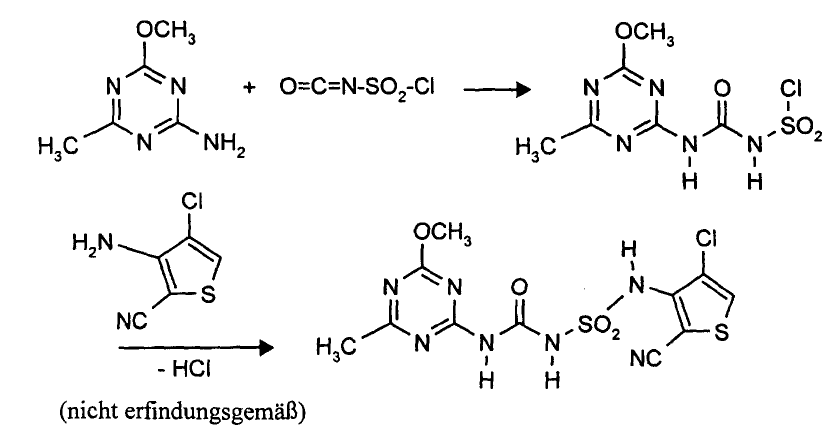

Die bei den erfindungsgemäßen Verfahren (a) (c) und (d) zur Herstellung der Verbindungen der allgemeinen Formel (I) als Ausgangsstoffe zu verwendenden Aminoazine sind durch die Formel (II) allgemein definiert. In der Formel (II) haben A, R¹, R² und R³ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, R¹, R² und R³ angegeben wurden.

Die Aminoazine der Formel (II) sind bekannte, zum Teil im Handel erhältliche Synthesechemikalien.

Die beim erfindungsgemäßen Verfahren (a) weiter als Ausgangsstoffe zu verwendenden Thienylsulfonyliso(thio)cyanate sind durch die Formel (III) allgemein definiert. In der Formel (III) haben Q, R⁴, R⁵ und R⁶ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q, R⁴, R⁵ und R⁶ angegeben wurden.

Die Ausgangsstoffe der Formel (III) sind bekannt und/oder können nach an sich bekannten Verfahren hergestellt werden (vgl. EP 30142) bzw. sind Gegenstand einer vorgängigen, nicht vorveröffentlichten Anmeldung (vgl. DE 19650196.2 vom 4. 12. 1996 / "Le A 32 173").

Man erhält die Thienylsulfonyliso(thio)cyanate der Formel (III), wenn man Thiophensulfonamide der allgemeinen Formel (V) - oben - mit Phosgen bzw. Thiophosgen, gegebenenfalls in Gegenwart eines Alkylisocyanats, wie z.B. Butylisocyanat, gegebenenfalls in Gegenwart eines Reaktionshilfsmittels, wie z.B. Diazabicyclo[2.2.2]octan, und in Gegenwart eines Verdünnungsmittels, wie z.B. Toluol, Xylol oder Chlorbenzol, bei Temperaturen zwischen 80°C und 150°C umsetzt und nach Ende der Umsetzung die flüchtigen Komponenten unter vermindertem Druck abdestilliert (vgl. die Herstellungsbeispiele).

Die beim erfindungsgemäßen Verfahren (b) zur Herstellung der Verbindungen der Formel (I) als Ausgangsstoffe zu verwendenden substituierten Aminoazine sind durch die Formel (IV) allgemein definiert. In der Formel (IV) haben A, Q, R¹, R² und R³ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, Q, R¹, R² und R³ angegeben wurden; Z steht vorzugsweise für Fluor, Chlor, Brom, C₁-C₄-Alkoxy oder Phenoxy, insbesondere für Chlor, Methoxy, Ethoxy oder Phenoxy.

Die Ausgangsstoffe der Formel (IV) sind bekannt und/oder können nach an sich bekannten Verfahren hergestellt werden (vgl. US 4690707, DE 19501174, Herstellungsbeispiele).

Die beim erfindungsgemäßen Verfahren (b) weiter als Ausgangsstoffe zu verwendenden Thiophensulfonamide sind durch die Formel (V) allgemein definiert. In der Formel (V) haben R⁴, R⁵ und R⁶ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R⁴, R⁵ und R⁶ angegeben wurden.

Die Ausgangsstoffe der Formel (V) sind bekannt und/oder können nach an sich bekannten Verfahren hergestellt werden (vgl. EP 30142).

Die beim erfindungsgemäßen Verfahren (c) zur Herstellung der Verbindungen der Formel (I) als Ausgangsstoffe zu verwendenden substituierten Thiophensulfon-(thio)amide sind durch die Formel (VI) allgemein definiert. In der Formel (VI) haben Q, R⁴, R⁵ und R⁶ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q, R⁴, R⁵ und R⁶ angegeben wurden; Z steht vorzugsweise für Fluor, Chlor, Brom, C₁-C₄-Alkoxy oder Phenoxy, insbesondere für Chlor, Methoxy, Ethoxy oder Phenoxy.

Die Ausgangsstoffe der Formel (VI) sind bekannt und/oder können nach an sich bekannten Verfahren hergestellt werden.

Die beim erfindungsgemäßen Verfahren (d) zur Herstellung von Verbindungen der Formel (I) als Ausgangsstoffe zu verwendenden Aminothiophene sind durch die Formel (VIII) allgemein definiert. In der Formel (VIII) haben R⁴, R⁵ und R⁶ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R⁴, R⁵ und R⁶ angegeben wurden.

Die Ausgangsstoffe der Formel (VIII) sind bekannt und/oder können nach an sich bekannten Verfahren hergestellt werden (vgl. DE 3303388, US 5457085).

Als Verdünnungsmittel zur Durchführung der erfindungsgemäßen Verfahren (a), (b), (c) und (d) kommen vor allem inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydröfuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester; Sulfoxide, wie Dimethylsulfoxid.

Die erfindungsgemäßen Verfahren (a), (b), (c) und (d) werden vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetall- -hydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a), (b), (c) und (d) in einem größeren Bereich variiert werden. Im allgemeinen arbeitet man bei Temperaturen zwischen -20°C und +150°C, vorzugsweise zwischen 0°C und +120°C.

Die erfindungsgemäßen Verfahren (a), (b), (c) und (d) werden im allgemeinen unter Normaldruck durchgeführt. Es ist jedoch auch möglich, die erfindungsgemäßen Verfahren unter erhöhtem oder vermindertem Druck - im allgemeinen zwischen 0, 1 bar und 10 bar - durchzuführen.

Zur Durchführung der erfindungsgemäßen Verfahren (a), (b), (c) und (d) werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgerührt (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B, durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise

Acetochlor, Acifluorfen(-sodium), Aclonifen, Alachlor, Alloxydim(-sodium), Ametryne, Amidochlor, Amidosulfuron, Asulam, Atrazine, Azimsulfuron, Benazolin, Benfuresate, Bensulfuron(-methyl), Bentazon, Benzofenap, Benzoylprop(-ethyl), Bialaphos, Bifenox, Bromobutide, Bromofenoxim, Bromoxynil, Butachlor, Butylate, Cafenstrole, Carbetamide, Chlomethoxyfen, Chloramben, Chloridazon, Chlorimuron(ethyl), Chlornitrofen, Chlorsulfuron, Chlortoluron, Cinmethylin, Cinosulfuron, Clethodim, Clodinafop(-propargyl), Clomazone, Clopyralid, Clopyrasulfuron, Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Difenzoquat, Diflufenican, Dimefuron, Dimepiperate, Dimethachlor, Dimethametryn, Dimethenamid, Dinitramine, Diphenamid, Diquat, Dithiopyr, Diuron, Dymron, EPTC, Esprocarb, Ethalfluralin, Ethametsulfuron(-methyl), Ethofumesate, Ethoxyfen, Etobenzanid, Fenoxaprop(-ethyl), Flamprop(-isopropyl), Flamprop(-isopropyl-L), Flamprop(-methyl), Flazasulfuron, Fluazifop(-butyl), Flumetsulam, Flumiclorac(-pentyl), Flumioxazin, Flumipropyn, Fluometuron, Fluorochloridone, Fluoroglycofen(-ethyl), Flupoxam, Flupropacil, Flurenol, Fluridone, Fluroxypyr, Flurprimidol, Flurtamone, Fomesafen, Glufosinate(-ammonium), Glyphosate(-isopropylammonium), Halosafen, Haloxyfop(-ethoxyethyl), Hexazinone, Imazamethabenz(methyl), Imazamethapyr, Imazamox, Imazapyr, Imazaquin, Imazethapyr, Imazosulfuron, Ioxynil, Isopropalin, Isoproturon, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, Metolachlor, Metosulam, Metoxuron, Metribuzin, Metsulfuron(-methyl), Molinate, Monolinuron, Naproanilide, Napropamide, Neburon, Nicosulfuron, Norflurazon Orbencarb, Oryzalin, Oxadiazon, Oxyfluorfen, Paraquat, Pendimethalin, Phenmedipham, Piperophos, Pretilachlor, Primisulfuron(-methyl), Prometryn, Propachlor, Propanil, Propaquizafop, Propyzamide, Prosulfocarb, Prosulfuron, Pyrazolate, Pyrazosulfuron(-ethyl), Pyrazoxyfen, Pyributicarb, Pyridate, Pyrithiobac(-sodium), Quinchlorac, Quinmerac, Quizalofop(ethyl), Quizalofop(-p-tefuryl), Rimsulfuron, Sethoxydim, Simazine, Simetryn, Sulcotrione, Sulfentrazone, Sulfometuron(-methyl), Sulfosate, Tebutam, Tebuthiuron, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

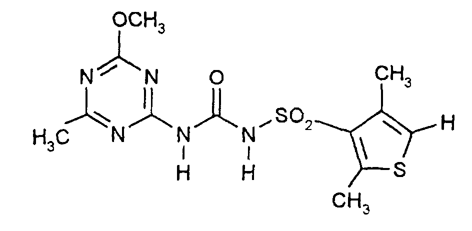

### (Verfahren (a))

1,7 g (7,8 mMol) 2,4-Dimethyl-thien-3-yl-sulfonyl-isocyanat werden bei Raumtemperatur (ca. 20°C) unter Rühren zu einer Mischung aus 1,01 g (7,8 mMol) 2-Amino-4-methoxy-6-methyl-1,3,5-triazin und 40 ml Acetonitril gegeben. Die Reaktionsmischung wird dann 12 Stunden unter Rückfluß erhitzt und auf Raumtemperatur abkühlen gelassen. Das kristallin angefallene Produkt wird dann durch Absaugen isoliert.

Man erhält 2,08 g (75% der Theorie) N-(4-Methoxy-6-methyl-1,3,5-triazin-2-yl)-N'-(2,4-dimethyl-thien-3-yl-sulfonyl)-harnstoff vom Schmelzpunkt 189°C.

### Beispiel 2

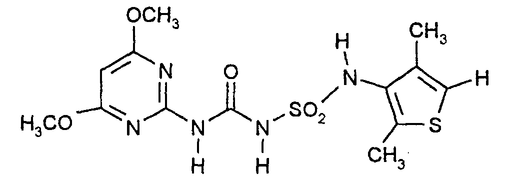

### (Verfahren (d))

2,78 g (19,7 mMol) Chlorsulfonylisocyanat werden in 200 ml Methylenchlorid gelöst und auf -10°C abgekühlt. Dann wird eine Lösung von 3,05 g (19,7 mMol) 2-Amino-4,6-dimethoxy-pyrimidin in 50 ml Methylenchlorid unter Rühren tropfenweise dazu gegeben und die Mischung wird 30 Minuten bei 0°C gerührt. Anschließend wird eine Lösung von 2,5 g (10,7 mMol) 3-Amino-2,4-dimethyl-thiophen und 2,0 g (20 mMol) Triethylamin in 100 ml Methylenchlorid tropfenweise dazu gegeben. Die Reaktionsmischung wird dann 15 Stunden bei Raumtemperatur (ca. 20°C) gerührt. Anschließend werden 100 ml 1N-Salzsäure dazu gegeben; die organische Phase wird abgetrennt, mit Wasser gewaschen, mit Magnesiumsulfat getrocknet und filtriert. Das Filtrat wird im Wasserstrahlvakuum eingeengt, der Rückstand mit Ethanol digeriert und das kristallin angefallene Produkt durch Absaugen isoliert.

Man erhält 4,4 g (58% der Theorie) N-(4,6-Dimethoxy-pyrimidin-2-yl)-N'-(2,4-dimethyl-thien-3-yl-amino-sulfonyl)-harnstoff vom Schmelzpunkt 190°C.

Analog zu den Herstellungsbeispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

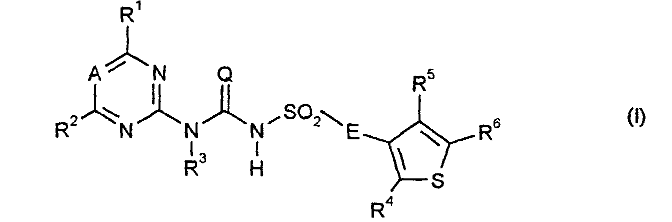

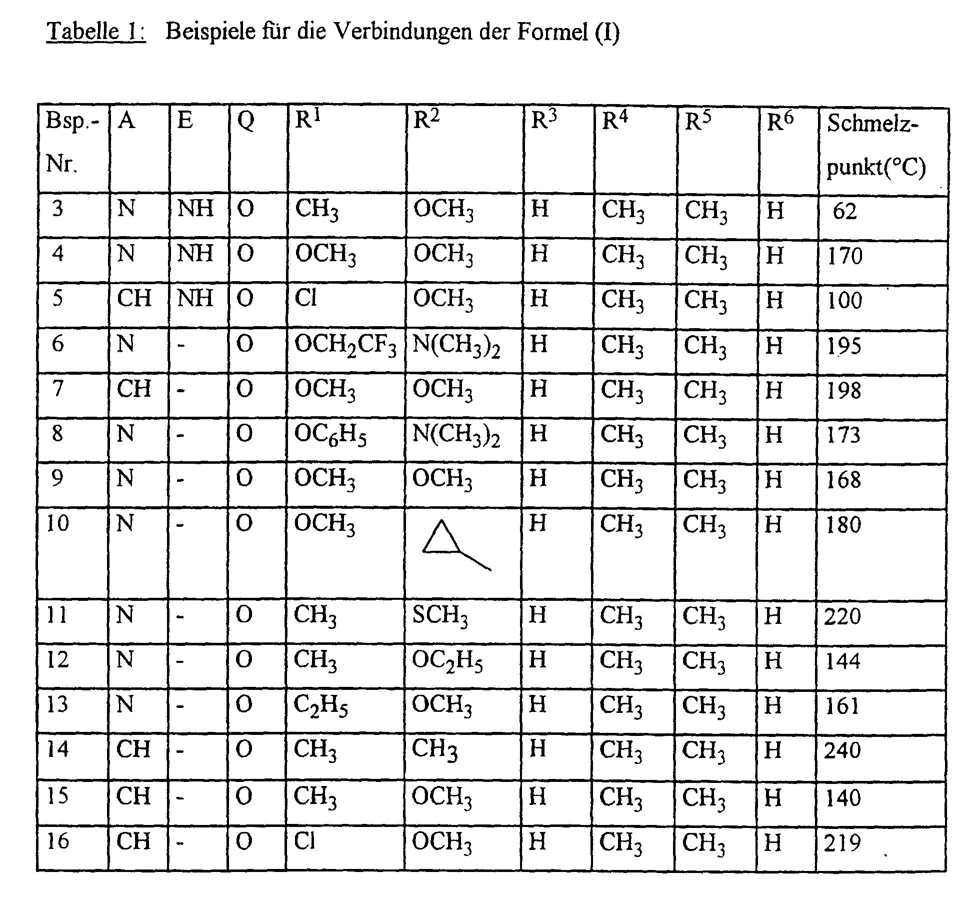

### Ausgangsstoffe der Formel (III):

### Beispiel (III-1)

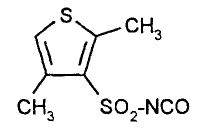

Eine Mischung aus 19,1 g (100 mMol) 2,4-Dimethyl-thiophen-3-sulfonamid, 10,0 g (100 mMol) Butylisocyanat und 100 ml Chloroform wird zum Sieden erhitzt und bei Rückflußtemperatur wird 4 Stunden lang Phosgen in die Mischung geleitet. Anschließend wird im Wasserstrahlvakuum eingeengt und der Rückstand einer Destillation im Ölpumpenvakuum unterworfen.

Man erhält 10,3 g (47% der Theorie) 2,4-Dimethyl-thien-3-yl-sulfonylisocyanat vom Siedebereich 135°C bis 140°C (bei 1 mbar).

### Ausgangsstoffe der Formel (V):

### Beispiel (V-1)

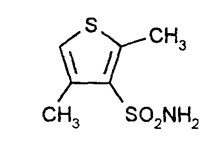

### Stufe 1

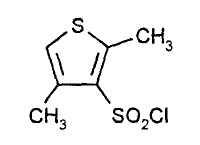

Eine Lösung von 13,9 g (109 mMol) 3-Amino-2,4-dimethyl-thiophen in 30 ml 10%iger Salzsäure wird auf 0°C abgekühlt und mit 50 ml konz. Salzsäure versetzt. Unter Kühlen auf 0°C bis -5°C wird dann unter Rühren eine Lösung von 8,6 g (125 mMol) Natriumnitrit in 22 ml Wasser tropfenweise dazu gegeben. Die Reaktionsmischung wird etwa eine Stunde lang bei 0°C bis -5°C gerührt. Anschließend wird überschüssiges Natriumnitrit mit Amidosulfonsäure zerstört. Die so erhaltene Diazoniumsalzlösung wird bei ca. 15°C zu einer Lösung von 12 g Schwefeldioxid in 100 ml 1,2-Dichlor-ethan tropfenweise gegeben. Dann werden 600 mg Kupfer(I)-chlorid und 600 mg Dodecyl-trimethylammoniumbromid dazu gegeben und die Reaktionsmischung wird etwa eine Stunde lang bei ca. 40°C und weitere 12 Stunden bei Raumtemperatur (ca. 20°C) gerührt Nach Zugabe von 6 g 30%iger Hydrogenperoxid-Lösung wird die Mischung weitere 30 Minuten gerührt. Die organische Phase wird dann abgetrennt, zweimal mit Wasser gewaschen, mit Magnesiumsulfat getrocknet und filtriert. Das Filtrat wird im Wasserstrahlvakuum eingeengt, der Rückstand mit Petrolether digeriert und das kristallin angefallene Produkt durch Absaugen isoliert.

Man erhält 9,6 g (42% der Theorie) 2,4-Dimethyl-thiophen-3-sulfochlorid vom Schmelzpunkt 79°C.

### Stufe 2

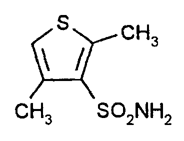

Eine Mischung aus 6,0 g (29 mMol) 2,4-Dimethyl-thiophen-3-sulfochlorid und 30 ml 25%iger wässriger Ammoniaklösung wird 12 Stunden bei Raumtemperatur (ca. 20°C) gerührt. Das hierbei kristallin angefallene Produkt wird dann durch Absaugen isoliert.

Man erhält 4,3 g (80% der Theorie) 2,4-Dimethyl-thiophen-3-sulfonamid vom Schmelzpunkt 135°C.

### Anwendungsbeispiele:

### Beispiel A

Pre-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung gespritzt, so daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 1, 6, 7, 8, 9, 10, 12, 14, 15 und 16 bei teilweise guter Verträglichkeit gegenüber Kulturpflanzen, wie z.B. Weizen und Gerste, sehr starke Wirkung gegen Unkräuter (vgl. Tabellen A-1 bis A-10); "ai." (active ingredient) = Wirkstoff.

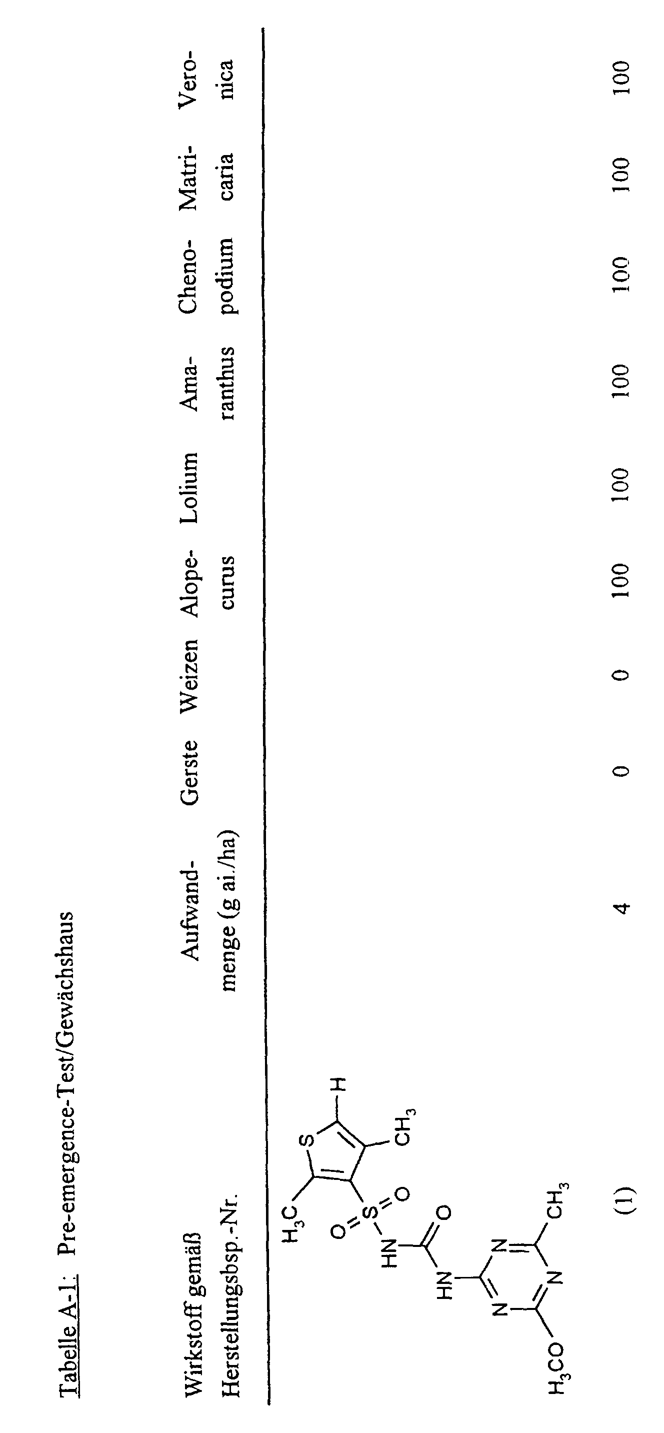

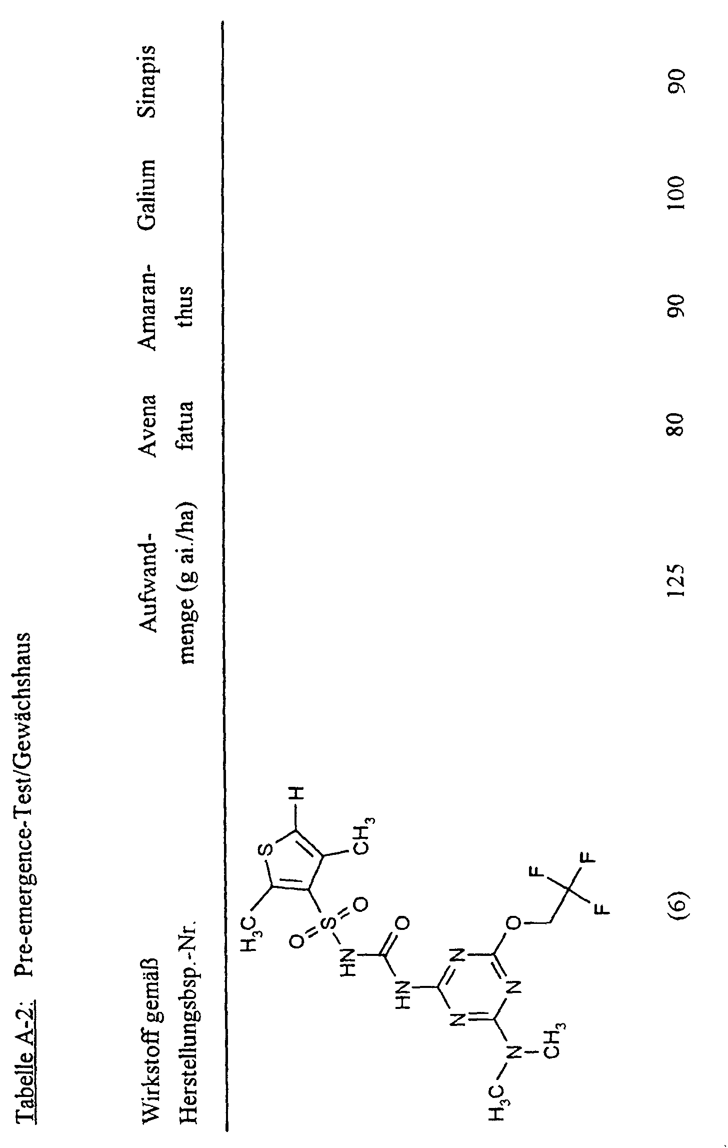

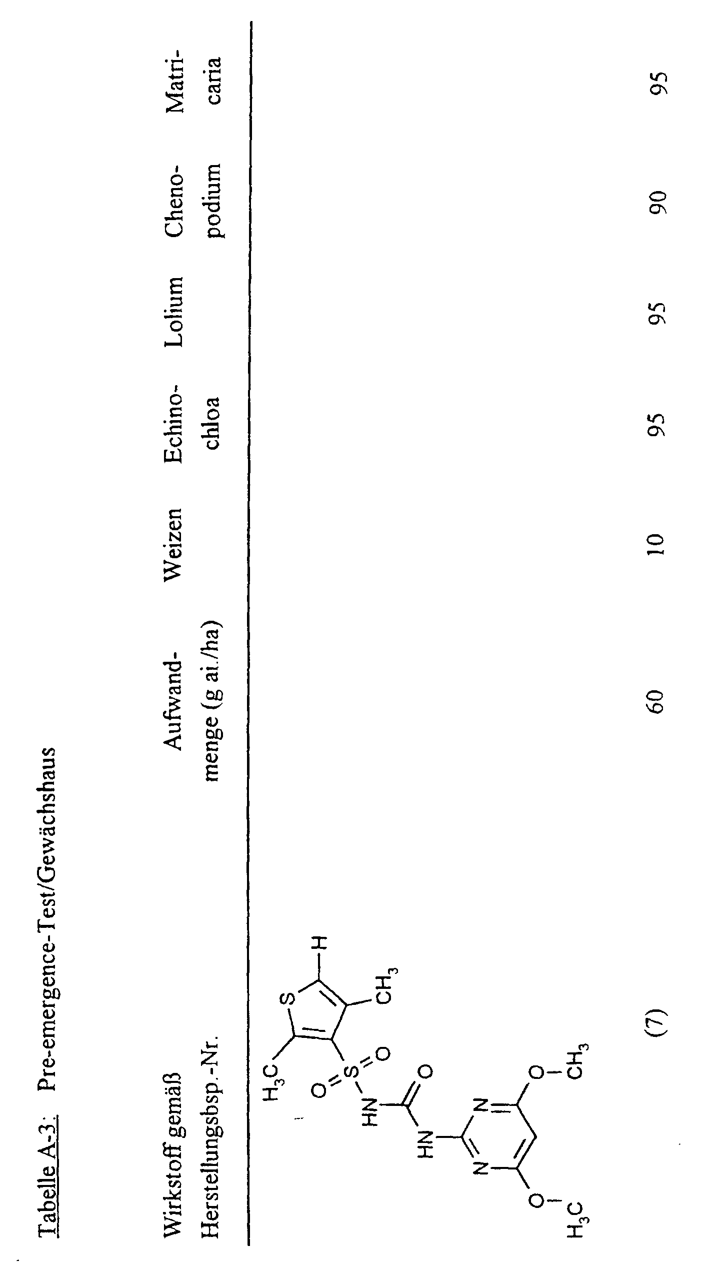

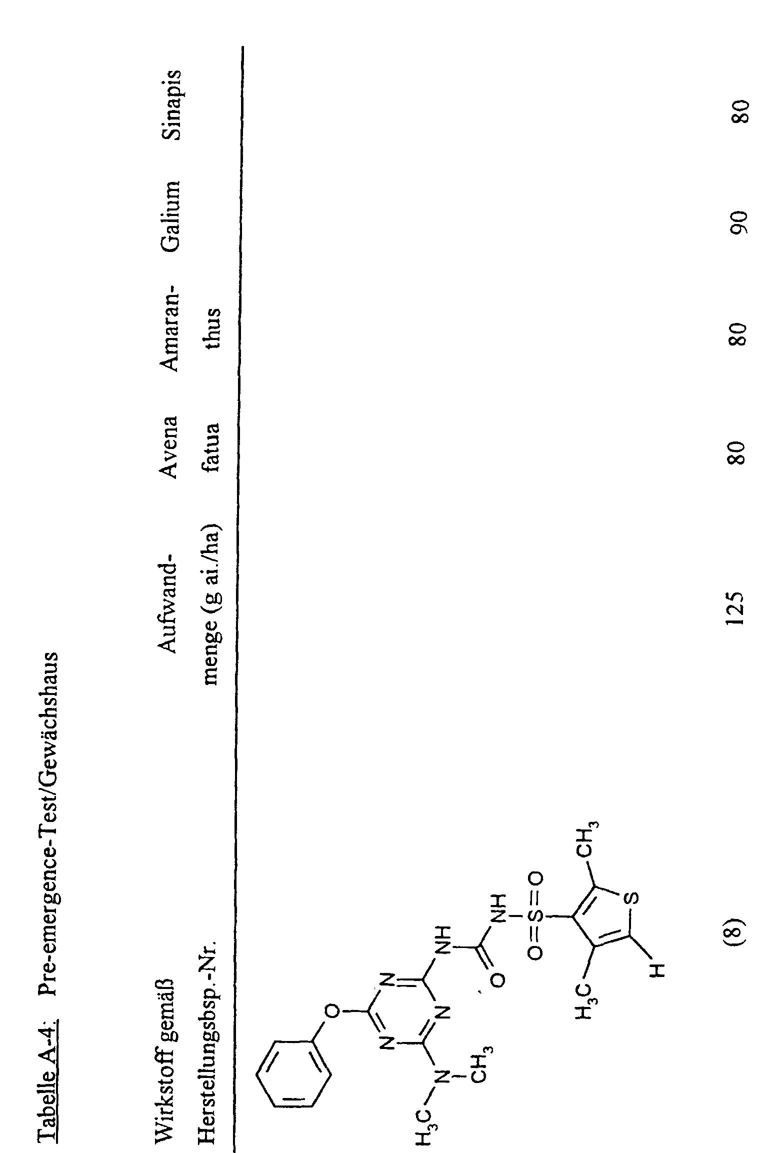

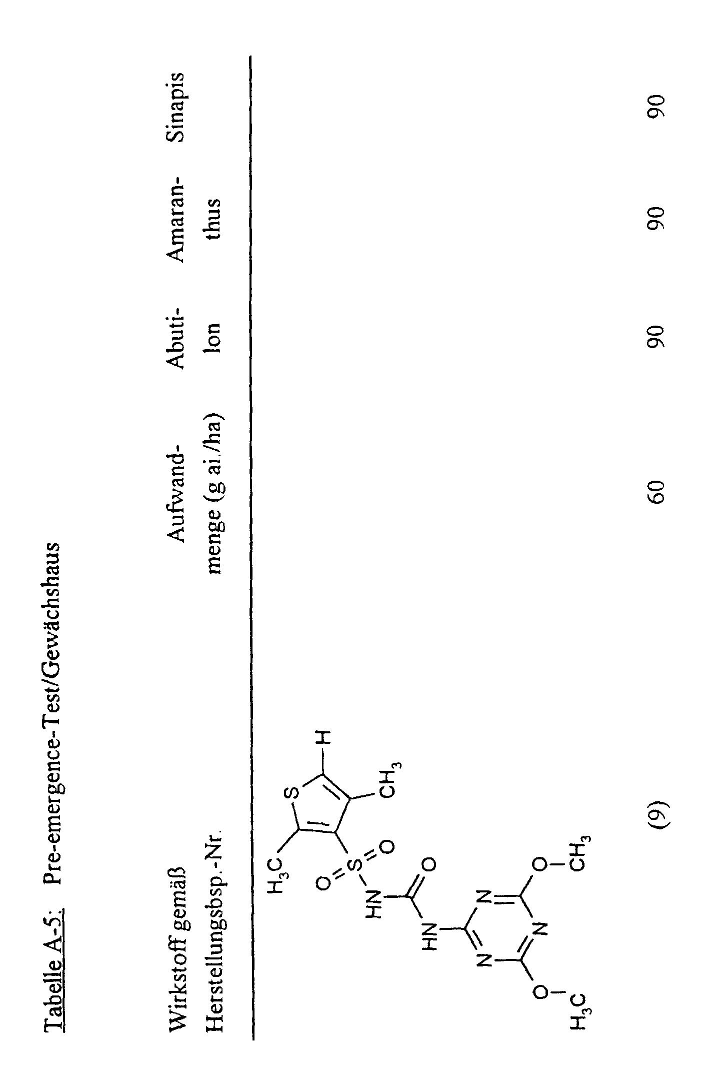

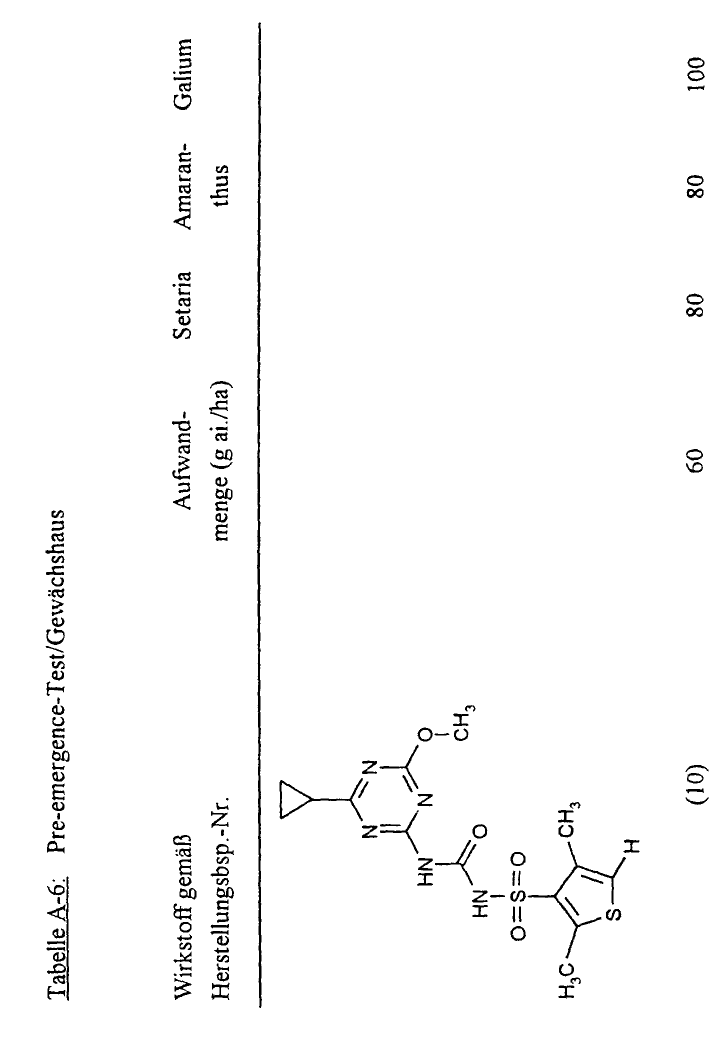

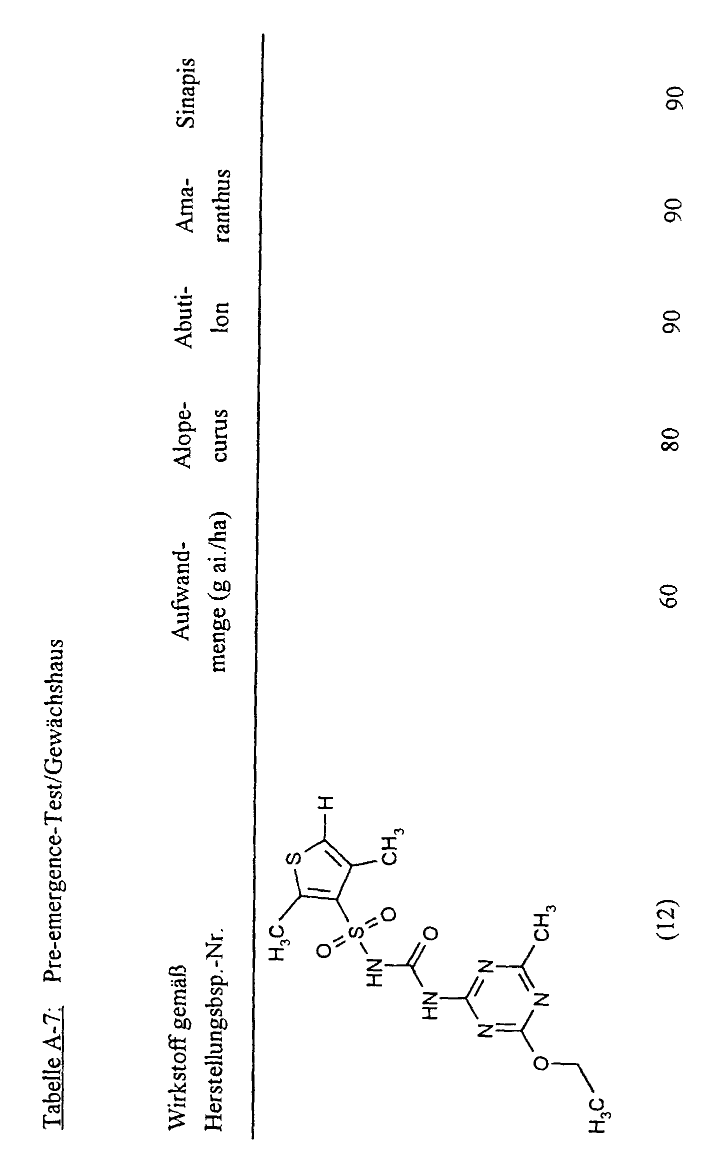

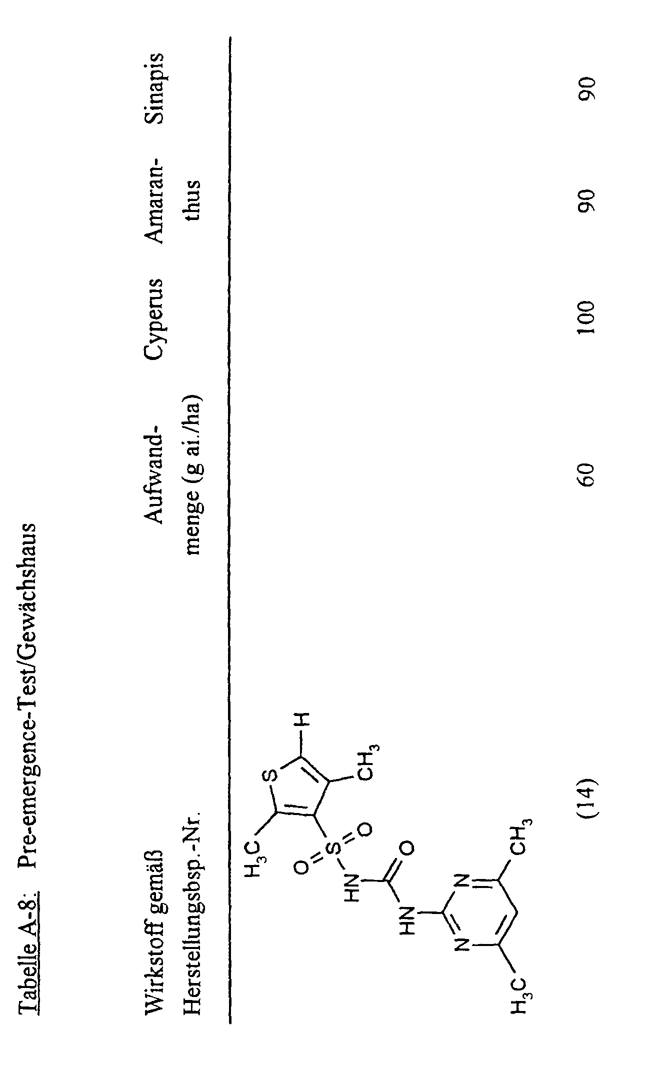

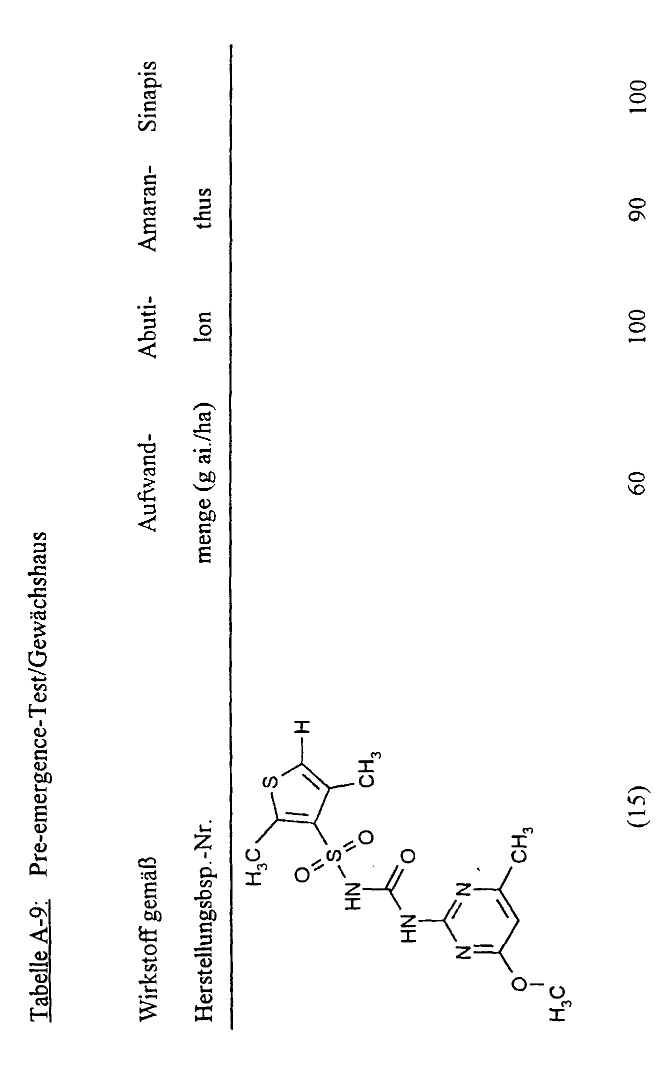

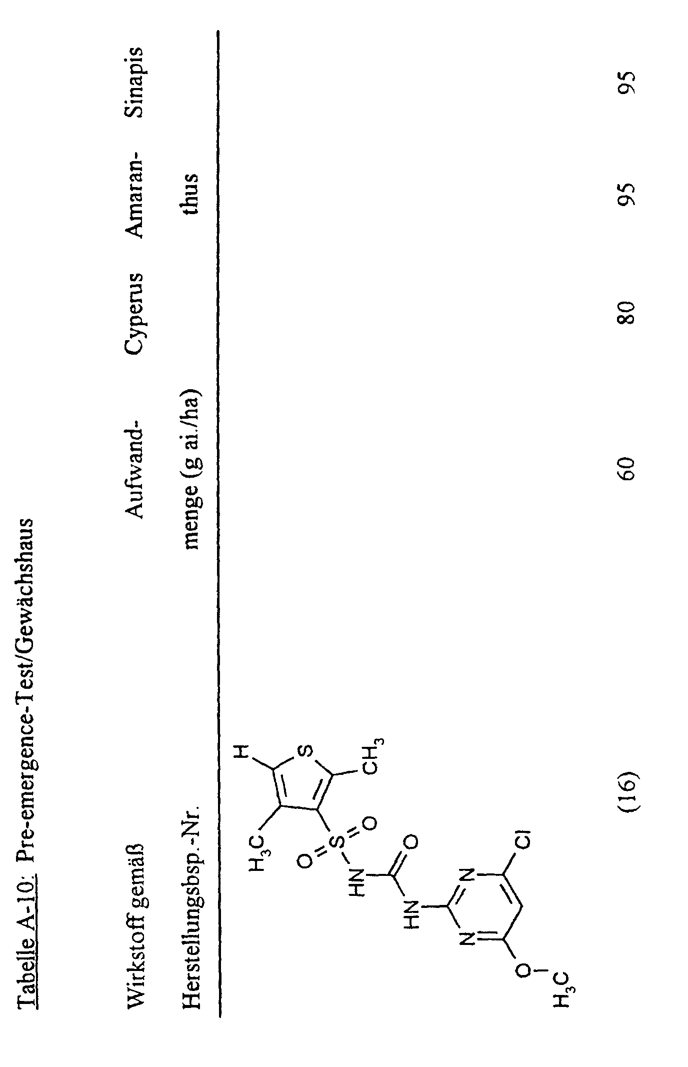

### Beispiel B

Post-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 1, 6, 7, 9, 11, 12, 13, 14, 15 und 16 sehr starke Wirkung gegen Unkräuter (vgl. Tabellen B-1 bis B-10).

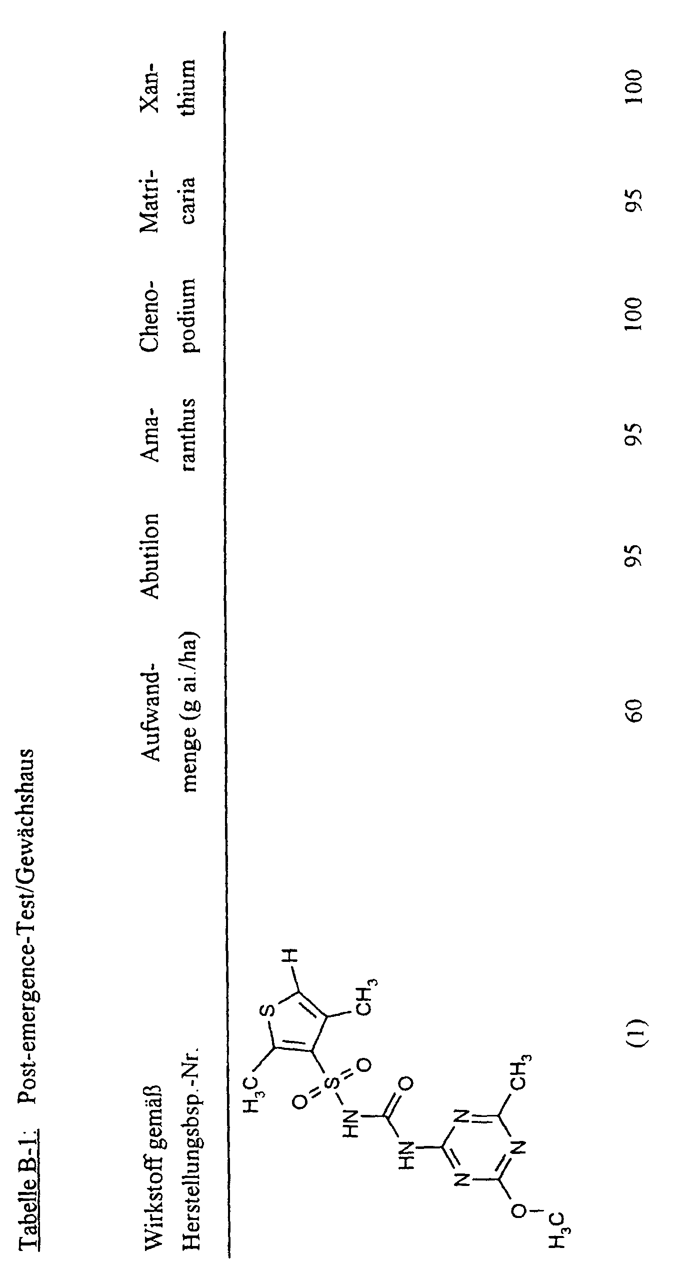

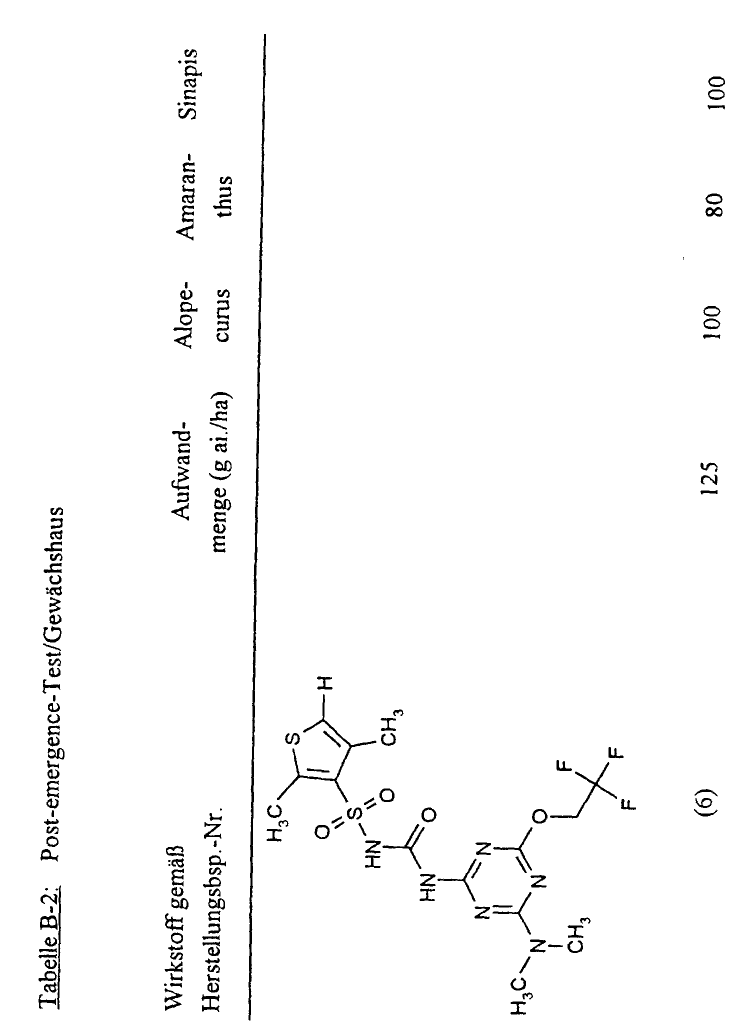

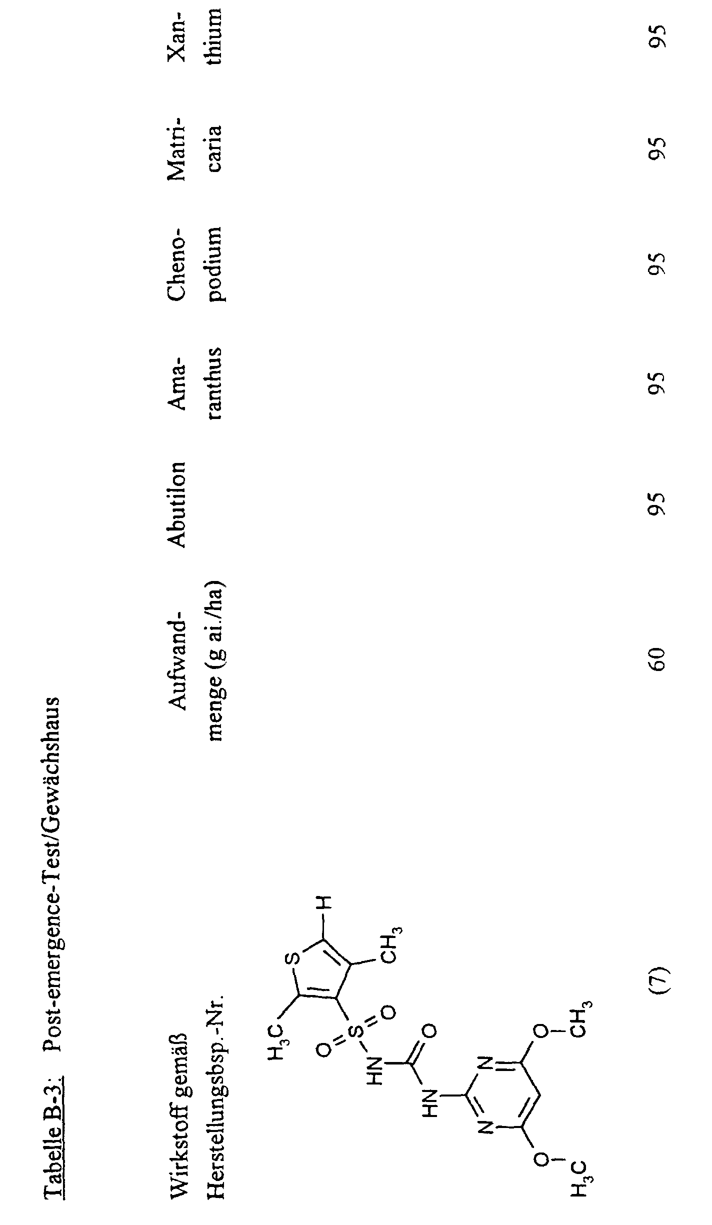

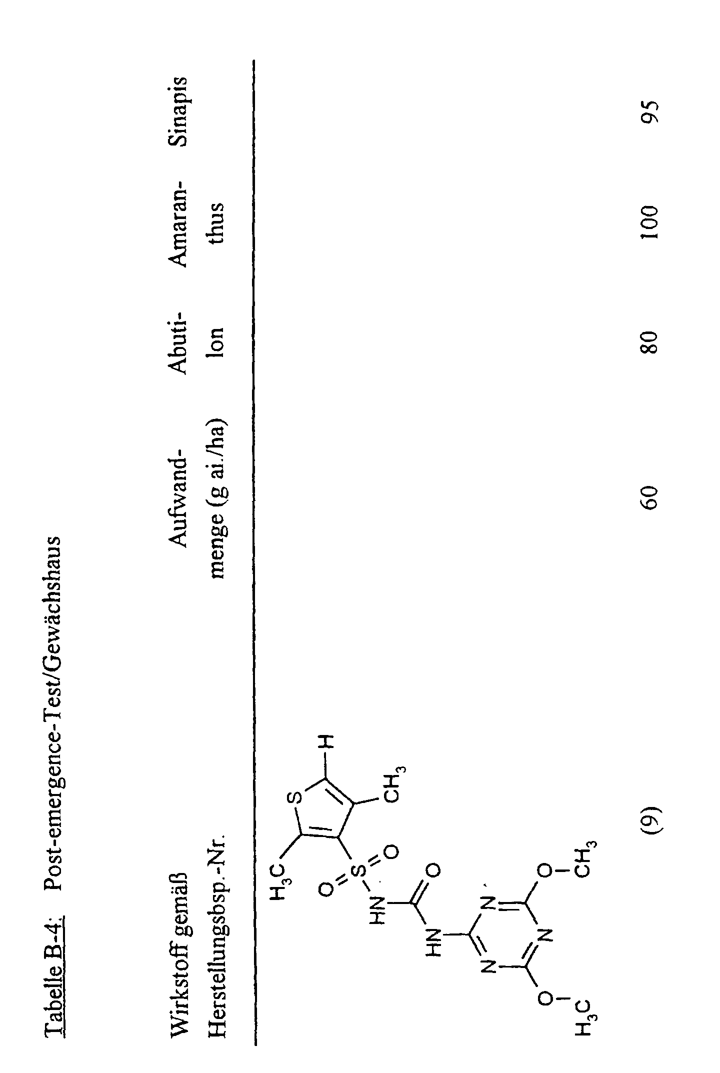

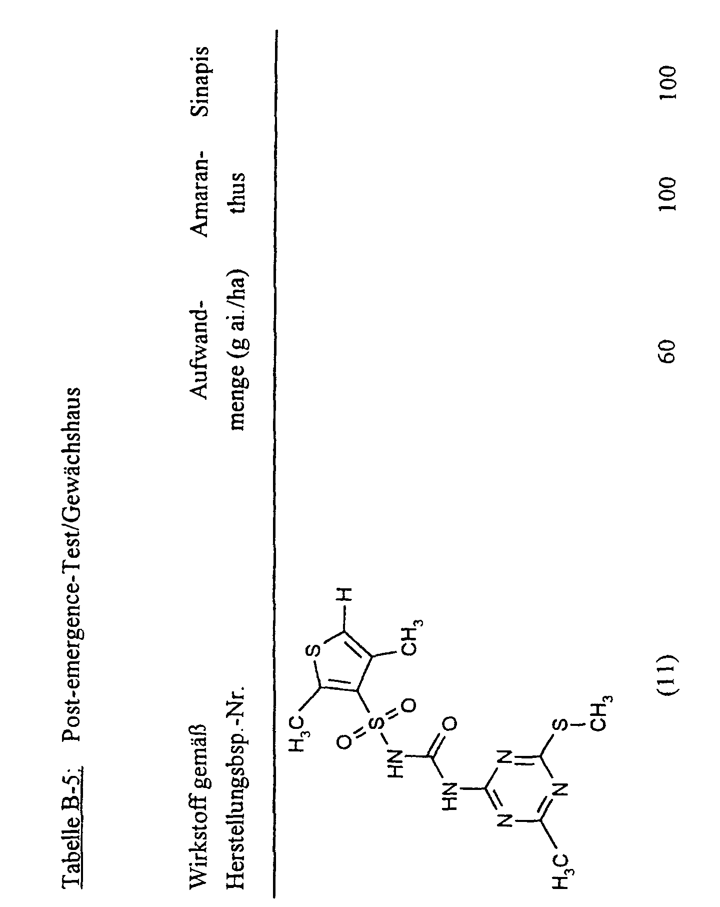

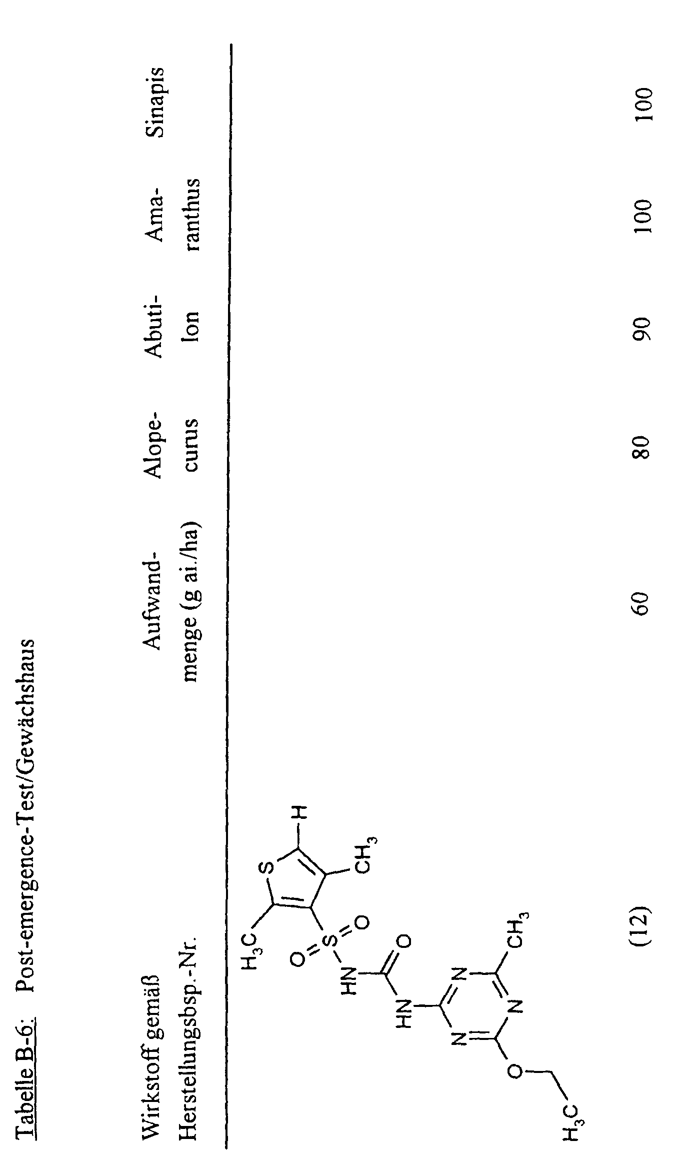

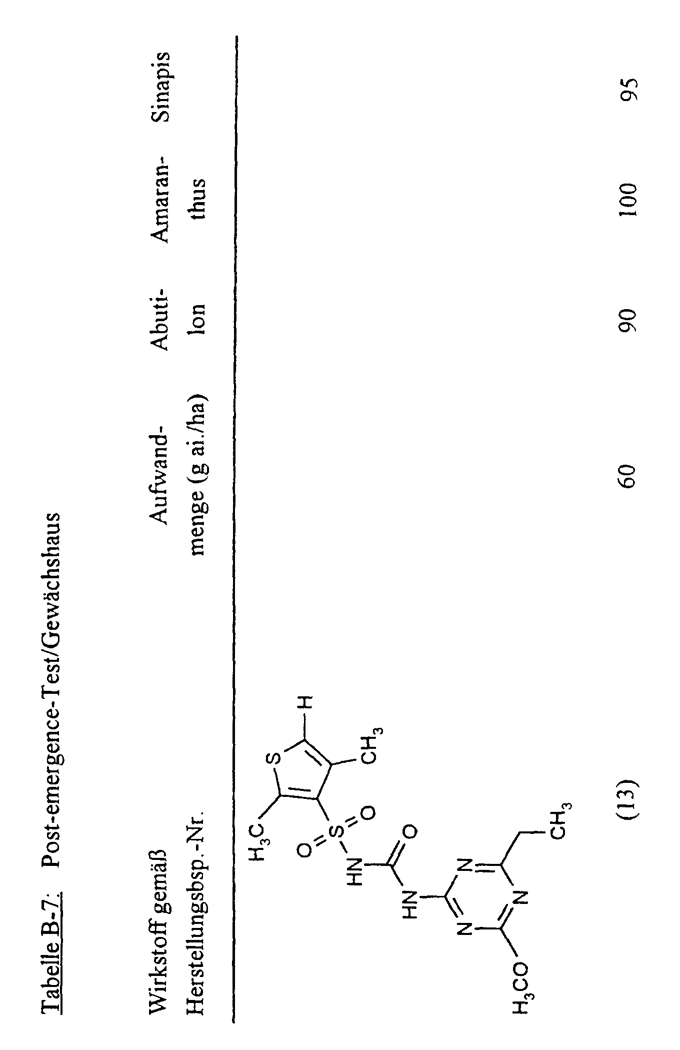

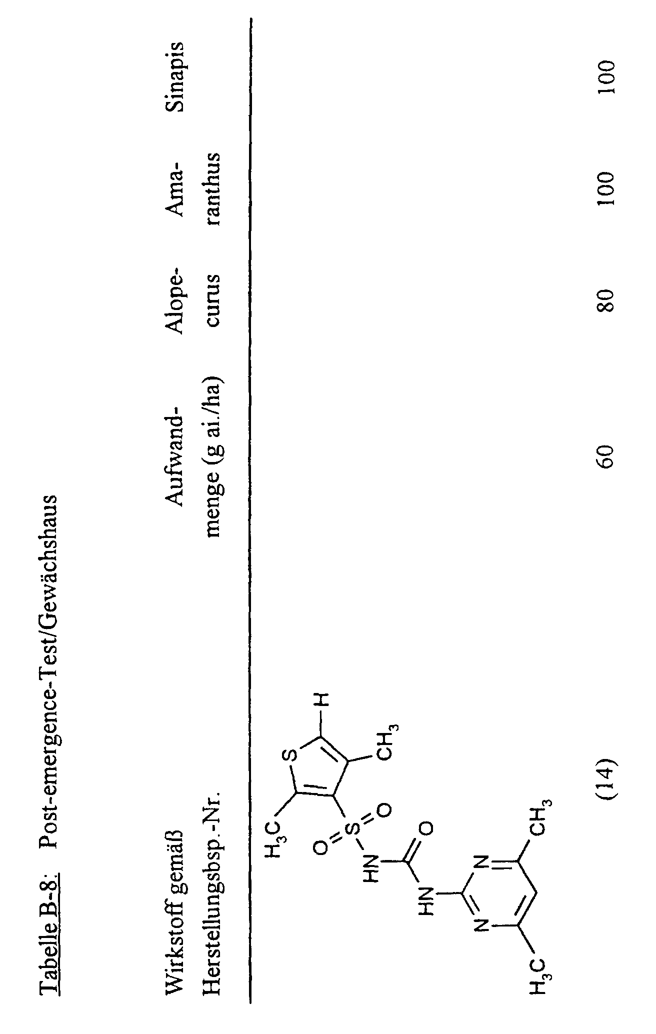

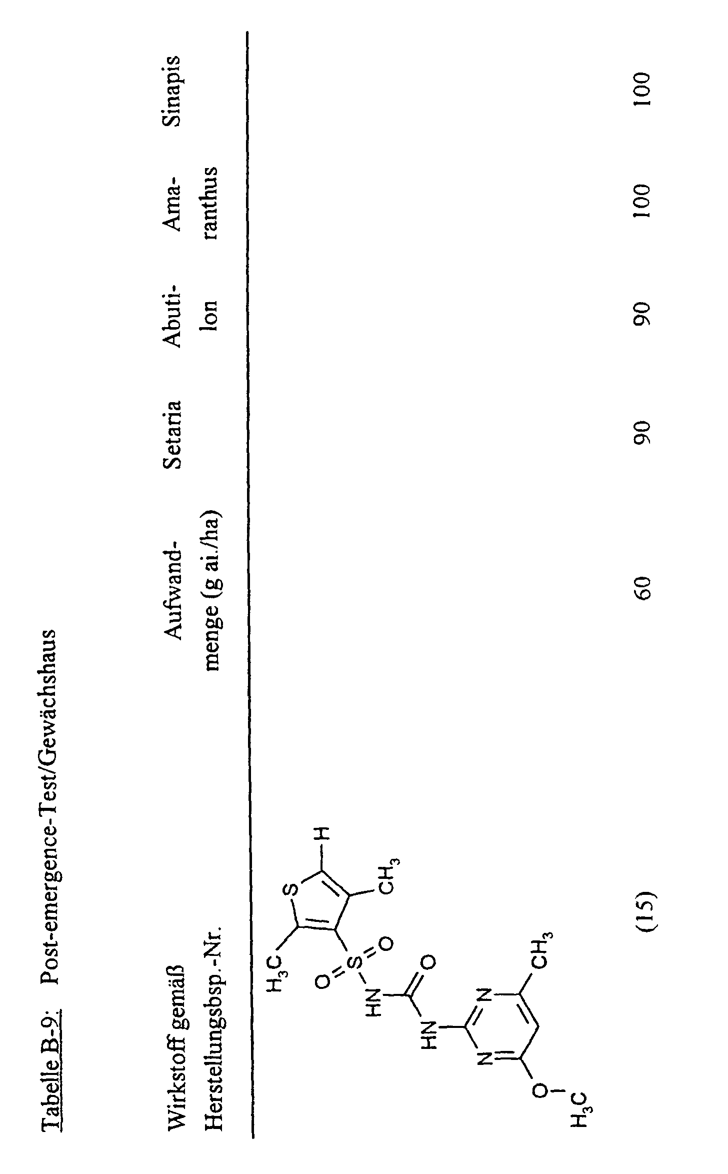

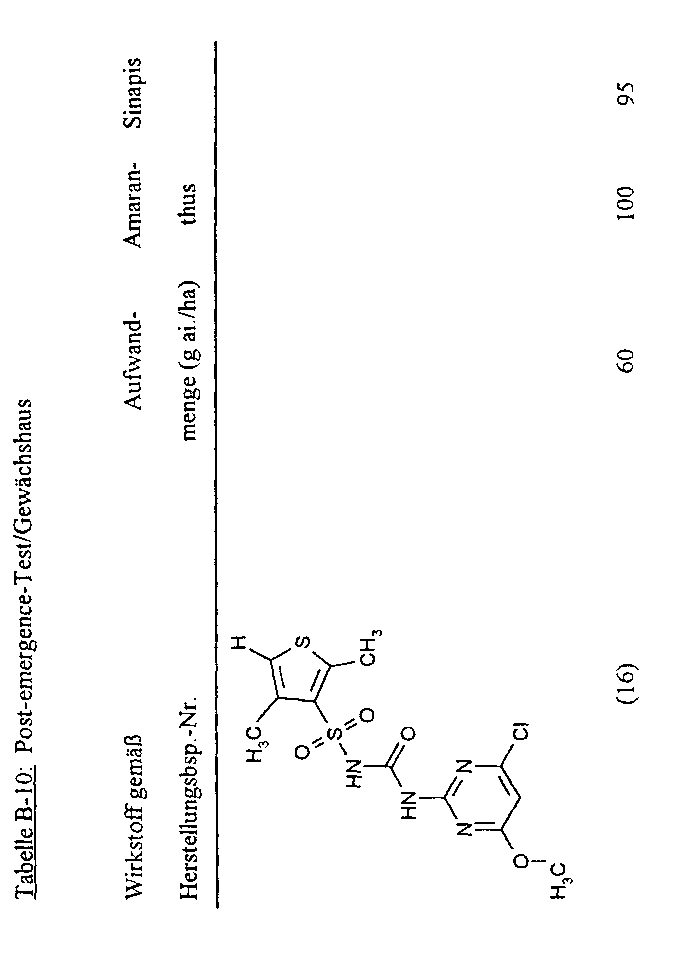

## Patentansprüche

1. Substituierte Thienyl(amino)sulfonyl(thio)harnstoffe der allgemeinen Formel (I) 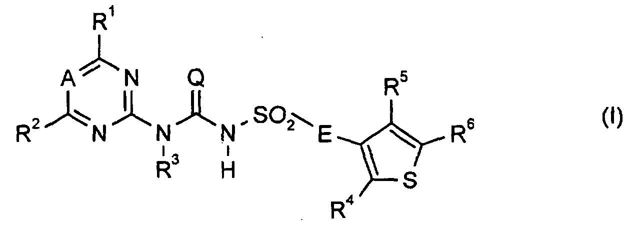 in welcher
A für Stickstoff oder eine CH-Gruppierung steht,
E für eine Einfachbindung oder eine NH-Gruppierung steht,
Q für Sauerstoff oder Schwefel steht,
R¹ für Wasserstoff, Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, für jeweils gegebenenfalls durch Cyano, Halogen, C₁-C₄-Alkyl oder C₁-C₄-Alkoxy substituiertes Cycloalkyl oder Cycloalkyloxy mit jeweils 3 bis 6 Kohlenstoffatomen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy steht,
R² für Wasserstoff oder Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, für jeweils gegebenenfalls durch Cyano, Halogen, C₁-C₄-Alkyl oder C₁-C₄-Alkoxy substituiertes Cycloalkyl oder Cycloalkyloxy mit jeweils 3 bis 6 Kohlenstoffatomen, oder für jeweils gegebenenfalls durch Cyano, Halogen, C₁-C₄-Alkyl oder C₁-C₄-Alkoxy substituiertes Phenoxy, Oxetanyloxy, Furyloxy oder Tetrahydrofuryloxy steht,
R³ für Wasserstoff oder gegebenenfalls durch C₁-C₄-Alkoxy, C₁-C₄-Alkyl-carbonyl oder C₁-C₄-Alkoxy-carbonyl substituiertes Alkyl mit 1 bis 4 Kohlenstoffatomen steht,
R⁴ für C₁-C₄-Alkyl steht,
R⁵ für C₁-C₄-Alkyl steht und
R⁶ für Wasserstoff, für Cyano, Halogen, für gegebenenfalls durch Cyano, Halogen oder C₁-C₄-Alkoxy substituiertes C₁-C₄-Alkyl, für jeweils gegebenenfalls durch Cyano oder Halogen substituiertes C₂-C₄-Alkenyl oder C₂-C₄-Alkinyl, für gegebenenfalls durch Cyano, Halogen oder C₁-C₄-Alkoxy substituiertes C₁-C₄-Alkoxy, oder für jeweils gegebenenfalls durch Cyano oder Halogen substituiertes C₂-C₄-Alkenyloxy oder C₂-C₄-Alkinyloxy steht,
sowie die Salze von Verbindungen der Formel (I).

2. Verbindungen der Formel (I) gemäß Anspruch 1, **dadurch gekennzeichnet, daß** darin
A für Stickstoff oder eine CH-Gruppierung steht,
E für eine Einfachbindung oder eine NH-Gruppierung steht,
Q für Sauerstoff oder Schwefel steht,
R¹ für Wasserstoff, Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R² für Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R³ für Wasserstoff oder gegebenenfalls durch Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl oder Ethyl steht,
R⁴ für Methyl, Ethyl, n- oder i-Propyl, n-, i- oder s-Butyl steht,
R⁵ für Methyl, Ethyl, n- oder i-Propyl, n-, i- oder s-Butyl steht und
R⁶ für Wasserstoff, für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methoxy oder Ethoxy substituiertes Methoxy, Ethoxy, n- oder i-Propoxy, n-, i-, s- oder t-Butoxy, oder für jeweils gegebenenfalls durch Cyano, Fluor oder Chlor substituiertes Propenyloxy, Butenyloxy, Propinyloxy oder Butinyloxy steht.

3. Verbindungen der Formel (I) gemäß Anspruch 1, **dadurch gekennzeichnet, daß** darin
A für Stickstoff oder eine CH-Gruppierung steht,
E für eine Einfachbindung steht,
Q für Sauerstoff oder Schwefel steht,
R¹ für Wasserstoff, Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R² für Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R³ für Wasserstoff oder gegebenenfalls durch Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl oder Ethyl steht,
R⁴ für Methyl, Ethyl, n- oder i-Propyl, n-, i- oder s-Butyl steht,
R⁵ für Methyl, Ethyl, n- oder i-Propyl, n-, i- oder s-Butyl steht und
R⁶ für Wasserstoff, für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methoxy oder Ethoxy substituiertes Methoxy, Ethoxy, n- oder i-Propoxy, n-, i-, s- oder t-Butoxy, oder für jeweils gegebenenfalls durch Cyano, Fluor oder Chlor substituiertes Propenyloxy, Butenyloxy, Propinyloxy oder Butinyloxy steht.

4. Verbindungen der Formel (I) gemäß Anspruch 1, **dadurch gekennzeichnet, daß** darin
A für Stickstoff oder eine CH-Gruppierung steht,
E für eine NH-Gruppierung steht,
Q für Sauerstoff oder Schwefel steht,
R¹ für Wasserstoff, Fluor, Chlor, Brom oder jeweils gegebenenfalls durch Cyano, Fluor; Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R² für Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R³ für Wasserstoff oder gegebenenfalls durch Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl oder Ethyl steht,
R⁴ für Methyl, Ethyl, n- oder i-Propyl, n-, i- oder s-Butyl steht,
R⁵ für Methyl, Ethyl, n- oder i-Propyl, n-, i- oder s-Butyl steht und
R⁶ für Wasserstoff, für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methoxy oder Ethoxy substituiertes Methoxy, Ethoxy, n- oder i-Propoxy, n-, i-, s- oder t-Butoxy, oder für jeweils gegebenenfalls durch Cyano, Fluor oder Chlor substituiertes Propenyloxy, Butenyloxy, Propinyloxy oder Butinyloxy steht.

5. Verfahren zur Herstellung von Verbindungen der Formel (I) gemäß Anspruch 1, **dadurch gekennzeichnet, daß** man
- für den Fall, daß in der allgemeinen Formel (I) E für eine Einfachbindung steht -
(a) Aminoazine der allgemeinen Formel (II) 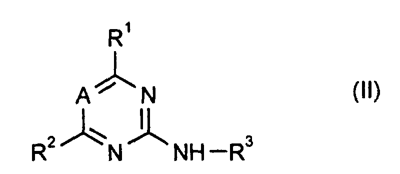 in welcher
A, R¹, R² und R³ die in Anspruch 1 angegebene Bedeutung haben,
mit Thienylsulfonyliso(thio)cyanaten der allgemeinen Formel (III) 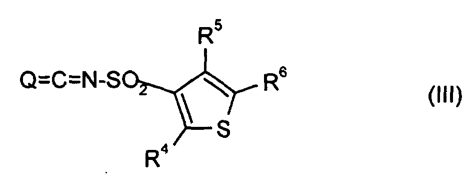 in welcher
Q, R⁴, R⁵ und R⁶ die in Anspruch 1 angegebene Bedeutung haben,
gegebenenfalls in Gegenwart eines Reaktionshilfsmittels und gegebenenfalls in Gegenwart eines Verdünnungsmittels umsetzt, oder
(b) substituierte Aminoazine der allgemeinen Formel (IV) 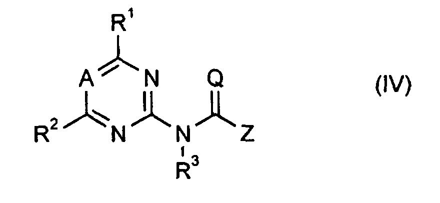 in welcher
A, Q, R¹, R² und R³ die in Anspruch 1 angegebene Bedeutung haben und
Z für Halogen, Alkoxy oder Aryloxy steht,
mit Thiophensulfonamiden der allgemeinen Formel (V) 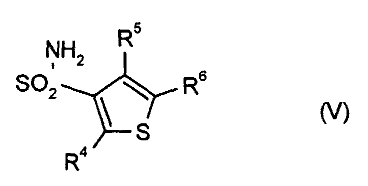 in welcher
R⁴, R⁵ und R⁶ die oben angegebene Bedeutung haben,
gegebenenfalls in Gegenwart eines Reaktionshilfsmittels und gegebenenfalls in Gegenwart eines Verdünnungsmittels umsetzt, oder
(c) Aminoazine der allgemeinen Formel (II) 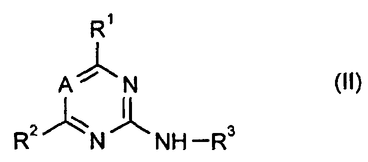 in welcher
A, R¹, R² und R³ die oben angegebene Bedeutung haben,
mit substituierten Thiophensulfon(thio)amiden der allgemeinen Formel (VI) 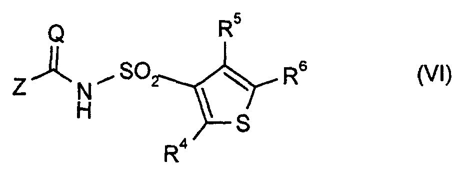 in welcher
Q, R⁴, R⁵ und R⁶ die oben angegebene Bedeutung haben und
Z für Halogen, Alkoxy oder Aryloxy steht,
gegebenenfalls in Gegenwart eines Reaktionshilfsmittels und gegebenenfalls in Gegenwart eines Verdünnungsmittels umsetzt,
oder daß man - für den Fall, daß E für eine NH-Gruppierung steht-
(d) Aminoazine der allgemeinen Formel (II) 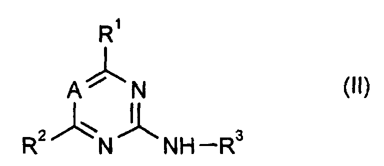 in welcher
A, R¹, R² und R³ die oben angegebene Bedeutung haben,
mit Chlorsulfonyliso(thio)cyanat, gegebenenfalls in Gegenwart eines Verdünnungsmittels, umsetzt und die hierbei gebildeten Chlorsulfonylharnstoffe der allgemeinen Formel (VII) 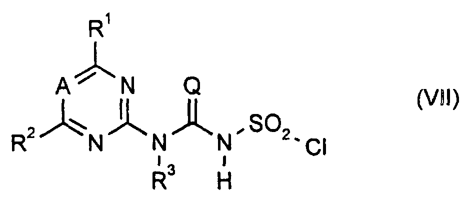 in welcher
A, Q, R¹, R² und R³ die oben angegebene Bedeutung haben,
mit Aminothiophenen der allgemeinen Formel (VIII) 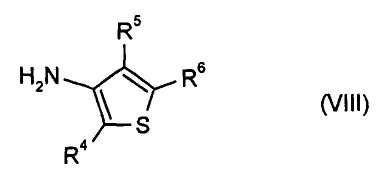 in welcher
R⁴, R⁵ und R⁶ die oben angegebene Bedeutung haben,
gegebenenfalls in Gegenwart eines Reaktionshilfsmittels und gegebenenfalls in Gegenwart eines Verdünnungsmittels umsetzt,
und gegebenenfalls die nach den Verfahren (a), (b), (c) oder (d) erhaltenen Verbindungen der Formel (I) nach üblichen Methoden in Salze überführt.

6. Herbizide Mittel, **gekennzeichnet durch** einen Gehalt an mindestens einer Verbindung der Formel (I) oder einem ihrer Salze gemäß Anspruch 1.

7. Verwendung von Verbindungen der allgemeinen Formel (I) oder deren Salzen gemäß Anspruch 1 zur Bekämpfung von unerwünschtem Pflanzenwachstum.

8. Verfahren zur Bekämpfung von Unkräutern, **dadurch gekennzeichnet, daß** man Verbindungen der allgemeinen Formel (I) oder deren Salze gemäß Anspruch 1 auf die Unkräuter oder ihren Lebensraum einwirken läßt.

9. Verfahren zur Herstellung von herbiziden Mitteln, **dadurch gekennzeichnet, daß** man Verbindungen der allgemeinen Formel (I) oder deren Salze gemäß Anspruch 1 mit Streckmitteln und/oder oberflächenaktiven Mitteln vermischt.

## Claims

1. Substituted thienyl(amino)sulphonyl(thio)ureas of the general formula (I) 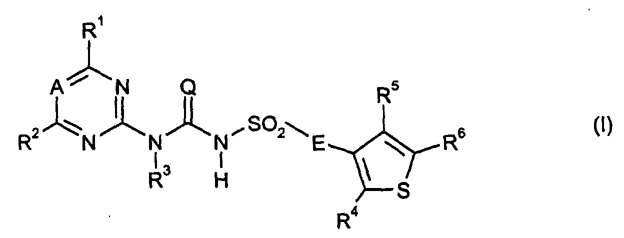 in which
A represents nitrogen or a CH grouping,
E represents a single bond or an NH grouping,
Q represents oxygen or sulphur,
R¹ represents hydrogen, halogen, represents in each case optionally cyano-, halogen- or C₁-C₄-alkoxy-substituted alkyl, alkoxy, alkylthio, alkylamino or dialkylamino having in each case 1 to 4 carbon atoms in the alkyl groups, represents in each case optionally cyano-, halogen-, C₁-C₄-alkyl- or C₁-C₄-alkoxy-substituted cycloalkyl or cycloalkyloxy having in each case 3 to 6 carbon atoms, or represents in each case optionally cyano-, halogen-, C₁-C₄-alkyl- or C₁-C₄-alkoxy-substituted phenoxy, oxetanyloxy, furyloxy or tetrahydrofuryloxy,
R² represents hydrogen or halogen, represents in each case optionally cyano-, halogen- or C₁-C₄-alkoxy-substituted alkyl, alkoxy, alkylthio, alkylamino or dialkylamino having in each case 1 to 4 carbon atoms in the alkyl groups, represents in each case optionally cyano-, halogen-, C₁-C₄-alkyl- or C₁-C₄-alkoxy-substituted cycloalkyl or cycloalkyloxy having in each case 3 to 6 carbon atoms, or represents in each case optionally cyano-, halogen-, C₁-C₄-alkyl- or C₁-C₄-alkoxy-substituted phenoxy, oxetanyloxy, furyloxy or tetrahydrofuryloxy,
R³ represents hydrogen or optionally C₁-C₄-alkoxy-, C₁-C₄-alkylcarbonyl- or C₁-C₄-alkoxy-carbonyl-substituted alkyl having 1 to 4 carbon atoms,
R⁴ represents C₁-C₄-alkyl,
R⁵ represents C₁-C₄-alkyl,
R⁶ represents hydrogen, represents cyano, halogen, represents optionally cyano-, halogen- or C₁-C₄-alkoxy-substituted C₁-C₄-alkyl, represents in each case optionally cyano- or halogen-substituted C₂-C₄-alkenyl or C₂-C₄-alkinyl, represents optionally cyano-, halogen- or C₁-C₄-alkoxysubstituted C₁-C₄-alkoxy, or represents in each case optionally cyano- or halogen-substituted C₂-C₄-alkenyloxy or C₂-C₄-alkinyloxy,
and the salts of compounds of the formula (I).

2. Compounds of the formula (I) according to Claim 1, **characterized in that**
A represents nitrogen or a CH grouping,
E represents a single bond or an NH grouping,
Q represents oxygen or sulphur,
R¹ represents hydrogen, fluorine, chlorine, bromine or in each case optionally cyano-, fluorine-, chlorine-, methoxy- or ethoxy-substituted methyl, ethyl, n- or i-propyl, methoxy, ethoxy, n- or i-propoxy, methylthio, ethylthio, n- or i-propylthio, methylamino, ethylamino, n- or i-propylamino, dimethylamino or diethylamino,
R² represents fluorine, chlorine, bromine or in each case optionally cyano-, fluorine-, chlorine-, methoxy- or ethoxy-substituted methyl, ethyl, n- or i-propyl, methoxy, ethoxy, n- or i-propoxy, methylthio, ethylthio, n- or i-propylthio, methylamino, ethylamino, n- or i- propylamino, dimethylamino or diethylamino,
R³ represents hydrogen or optionally methoxy-, ethoxy-, n- or i-propoxy-, acetyl-, propionyl, n- or i-butyroyl-, methoxycarbonyl-, ethoxy- carbonyl-, n- or i-propoxycarbonyl-substituted methyl or ethyl,
R⁴ represents methyl, ethyl, n- or i-propyl, n-, i- or s-butyl,
R⁵ represents methyl, ethyl, n- or i-propyl, n-, i- or s-butyl, and
R⁶ represents hydrogen, represents cyano, fluorine, chlorine, bromine, represents in each case optionally cyano-, fluorine-, chlorine-, methoxy- or ethoxy-substituted methyl, ethyl, n- or i-propyl, n-, i- or s- butyl, represents in each case optionally cyano-, fluorine- or chlorine-substituted propenyl, butenyl, propinyl or butinyl, represents in each case optionally cyano-, fluorine-, chlorine-, methoxy- or ethoxy- substituted methoxy, ethoxy, n- or i-propoxy, n-, i-, s- or t-butoxy, or represents in each case optionally cyano-, fluorine- or chlorine- substituted propenyloxy, butenyloxy, propinyloxy or butinyloxy.

3. Compounds of the formula (I) according to Claim 1, **characterized in that**
A represents nitrogen or a CH grouping,
E represents a single bond,
Q represents oxygen or sulphur,
R¹ represents hydrogen, fluorine, chlorine, bromine or in each case optionally cyano-, fluorine-, chlorine-, methoxy- or ethoxy-substituted methyl, ethyl, n- or i-propyl, methoxy, ethoxy, n- or i-propoxy, methylthio, ethylthio, n- or i-propylthio, methylamino, ethylamino, n- or i-propylamino, dimethylamino or diethylamino,
R² represents fluorine, chlorine, bromine or in each case optionally cyano-, fluorine-, chlorine-, methoxy- or ethoxy-substituted methyl, ethyl, n- or i-propyl, methoxy, ethoxy, n- or i-propoxy, methylthio, ethylthio, n- or i-propylthio, methylamino, ethylamino, n- or i- propylamino, dimethylamino or diethylamino,
R³ represents hydrogen or optionally methoxy-, ethoxy-, n- or i-propoxy-, acetyl-, propionyl-, n- or i-butyroyl-, methoxycarbonyl-, ethoxycarbonyl-, n- or i-propoxycarbonyl-substituted methyl or ethyl,
R⁴ represents methyl, ethyl, n- or i-propyl, n-, i- or s-butyl,
R⁵ represents methyl, ethyl, n- or i-propyl, n-, i- or s-butyl, and
R⁶ represents hydrogen, represents cyano, fluorine, chlorine, bromine, represents in each case optionally cyano-, fluorine-, chlorine-, methoxy- or ethoxy-substituted methyl, ethyl, n- or i-propyl, n-, i- or s- butyl, represents in each case optionally cyano-, fluorine- or chlorine- substituted propenyl, butenyl, propinyl or butinyl, represents in each case optionally cyano-, fluorine-, chlorine-, methoxy- or ethoxy- substituted methoxy, ethoxy, n- or i-propoxy, n-, i-, s- or t-butoxy, or represents in each case optionally cyano-, fluorine- or chlorine- substituted propenyloxy, butenyloxy, propinyloxy or butinyloxy.

4. Compounds of the formula (I) according to Claim 1, **characterized in that**
A represents nitrogen or a CH grouping,
E represents an NH grouping,
Q represents oxygen or sulphur,
R¹ represents hydrogen, fluorine, chlorine, bromine or in each case optionally cyano-, fluorine-, chlorine-, methoxy- or ethoxy-substituted methyl, ethyl, n- or i-propyl, methoxy, ethoxy, n- or i-propoxy, methylthio, ethylthio, n- or i-propylthio, methylamino, ethylamino, n- or i-propylamino, dimethylamino or diethylamino,
R² represents fluorine, chlorine, bromine or in each case optionally cyano-, fluorine-, chlorine-, methoxy- or ethoxy-substituted methyl, ethyl, n- or i-propyl, methoxy, ethoxy, n- or i-propoxy, methylthio, ethylthio, n- or i-propylthio, methylamino, ethylamino, n- or i- propylamino, dimethylamino or diethylamino,
R³ represents hydrogen or optionally methoxy-, ethoxy-, n- or i-propoxy-, acetyl-, propionyl-, n- or i-butyroyl-, methoxycarbonyl-, ethoxycarbonyl-, n- or i-propoxycarbonyl-substituted methyl or ethyl,
R⁴ represents methyl, ethyl, n- or i-propyl, n-, i- or s-butyl,
R⁵ represents methyl, ethyl, n- or i-propyl, n-, i- or s-butyl, and
R⁶ represents hydrogen, represents cyano, fluorine, chlorine, bromine, represents in each case optionally cyano-, fluorine-, chlorine-, methoxy- or ethoxy-substituted methyl, ethyl, n- or i-propyl, n-, i- or s- butyl, represents in each case optionally cyano-, fluorine- or chlorine- substituted propenyl, butenyl, propinyl or butinyl, represents in each case optionally cyano-, fluorine-, chlorine-, methoxy- or ethoxy- substituted methoxy, ethoxy, n- or i-propoxy, n-, i-, s- or t-butoxy, or represents in each case optionally cyano-, fluorine- or chlorine- substituted propenyloxy, butenyloxy, propinyloxy or butinyloxy.

5. Process for preparing compounds of formula (I) according to Claim 1, **characterized in that**
- in the case of E representing a single bond in the general formula (I) -
(a) aminoazines of the general formula (II) 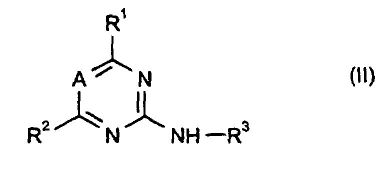 in which
A, R¹, R² and R³ are each as defined in Claim 1,
are reacted with thienylsulphonyl iso(thio)cyanates of the general formula (III) 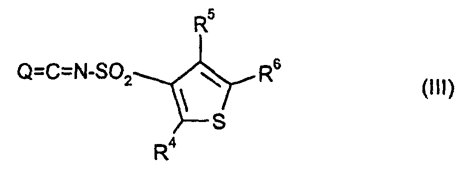 in which
Q, R⁴, R⁵ and R⁶ are each as defined in Claim 1,
if appropriate in the presence of a reaction auxiliary and if appropriate in the presence of a diluent, or
(b) substituted aminoazines of the general formula (IV) 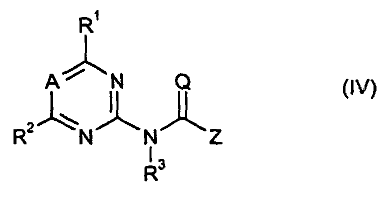 in which
A, Q, R¹, R² and R³ are each as defined in Claim 1 and
Z represents halogen, alkoxy or aryloxy,
are reacted with thiophenesulphonamides of the general formula (V) 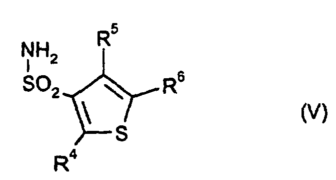 in which
R⁴, R⁵ and R⁶ are each as defined above,
if appropriate in the presence of a reaction auxiliary and if appropriate in the presence of a diluent, or
(c) aminoazines of the general formula (II) 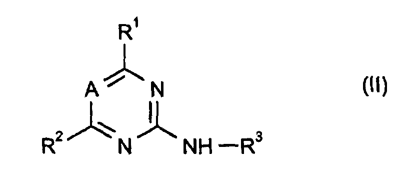 in which
A, R¹, R² and R³ are each as defined above,
are reacted with substituted thiophenesulphon(thio)amides of the general formula (VI) 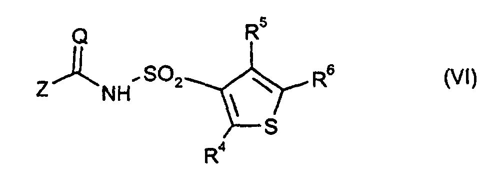 in which
Q, R⁴, R⁵ and R⁶ are each as defined above and
Z represents halogen, alkoxy or aryloxy,
if appropriate in the presence of a reaction auxiliary and if appropriate in the presence of a diluent,
or **in that** - in the case of E representing an NH grouping -
(d) aminoazines of the general formula (II) 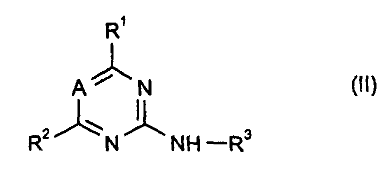 in which
A, R¹, R² and R³ are each as defined above
are reacted with chlorosulphonyl iso(thio)cyanate, if appropriate in the presence of a diluent, and the resulting chlorosulphonylureas of the general formula (VII) 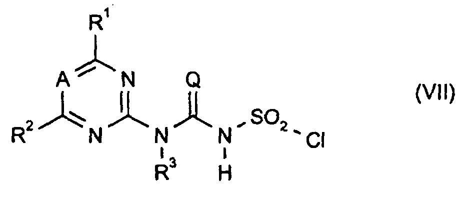 in which
A, Q, R¹, R² and R³ are each as defined above
are reacted with aminothiophenes of the general formula (VIII) 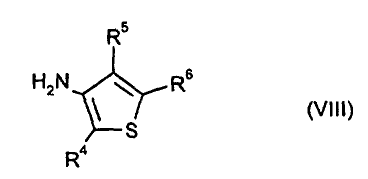 in which
R⁴, R⁵ and R⁶ are each as defined above,
if appropriate in the presence of a reaction auxiliary and if appropriate in the presence of a diluent,
and the compounds of the formula (I) obtained by processes (a), (b), (c) or (d) are optionally converted into salts by customary methods.

6. Herbicidal compositions, **characterized in that** they comprise at least one compound of the formula (I) or one of its salts according to Claim 1.

7. The use of compounds of the general formula (I) or salts thereof according to Claim 1 for controlling undesirable plant growth.

8. Method for controlling weeds, **characterized in that** compounds of the general formula (I) or salts thereof according to Claim 1 are allowed to act on the weeds or their habitat.

9. Process for preparing herbicidal compositions, **characterized in that** compounds of the general formula (I) or salts thereof according to Claim 1 are mixed with extenders and/or surfactants.

## Revendications

1. Thiényl(amino)sulfonyl(thio)urées substituées de formule générale (I) 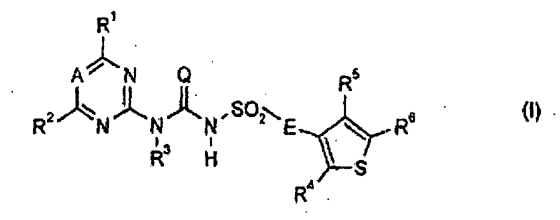 dans laquelle
A représente l'azote ou un groupement CH,
E est une liaison simple ou un groupement NH,
Q représente l'oxygène ou le soufre,
R¹ représente l'hydrogène, un halogène, un reste alkyle, alkoxy, alkylthio, alkylamino ou dialkylamino ayant chacun 1 à 4 atomes de carbone dans les groupes alkyle et chacun étant éventuellement substitué par, un radical cyano, halogène ou alkoxy en C₁ à C₄, un reste cycloalkyle ou cycloalkoxy ayant chacun 3 à 6 atomes de carbone et chacun étant éventuellement substitué par un radical cyano, halogéno, alkyle en C₁ à C₄ ou alkoxy en C₁ à C₄, ou un reste phénoxy, oxétanyloxy, furyloxy ou tétrahydrofuryloxy, chacun éventuellement substitué par un radical cyano, halogène, alkyle en C₁ à C₄ ou alkoxy en C₁ à C₄,
R² représente l'hydrogène ou un halogène, un reste alkyle, alkoxy, alkylthio, alkylamino ou dialkylamino ayant chacun 1 à 4 atomes de carbone dans les groupes alkyle et chacun éventuellement substitué par un radical cyano, halogène ou alkoxy en C₁ à C₄, un reste cycloalkyle ou cycloalkyloxy ayant chacun 3 à 6 atomes de carbone et chacun étant éventuellement substitué par un radical cyano, halogène, alkyle en C₁ à C₄ ou alkoxy en C₁ à C₄, ou un reste phénoxy, oxétanyloxy, furyloxy ou tétrahydrofuryloxy, chacun éventuellement substitué par un radical cyano, halogène, alkyle en C₁ à C₄ ou alkoxy en C₁ à C₄,
R³ représente l'hydrogène ou un reste alkyle ayant 1 à 4 atomes de carbone éventuellement substitué par un radical alkoxy en C₁ à C₄, (alkyle en C₁ à C₄) - carbonyle ou (alkoxy en C₁ à C₄)-carbonyle,
R⁴ est un reste alkyle en C₁ à C₄,
R⁵ est un reste alkyle en C₁ à C₄ et
R⁶ représente l'hydrogène, un groupe cyano, un halogène, un reste alkyle en C₁ à C₄ éventuellement substitué par un radical cyano, halogène ou alkoxy en C₁ à C₄, un reste alcényle en C₂ à C₄ ou alcynyle en C₂ à C₄, chacun éventuellement substitué par un radical cyano ou halogène, un reste alkoxy en C₁ à C₄ éventuellement substitué par un radical cyano, halogène ou alkoxy en C₁ à C₄, ou un reste alcényloxy en C₂ à C₄ ou alcynyloxy en C₂ à C₄, chacun éventuellement substitué par un radical cyano ou halogéno,
ainsi que les sels de composés de formule (I).

2. Composés de formule (I) suivant la revendication 1, **caractérisés en ce que** dans la formule
A représente l'azote ou un groupement CH,
E est une liaison simple ou un groupement NH,
Q est l'oxygène ou le soufre,
R¹ est l'hydrogène, le fluor, le chlore, le brome ou un reste méthyle, éthyle, n-propyle, isopropyle, méthoxy, éthoxy, n-propoxy, isopropoxy, méthylthio, éthylthio, n-propylthio, isopropylthio, méthylamino, éthylamino, n-propylamino, isopropylamino, diméthylamino ou diéthylamino, chacun éventuellement substitué par un radical cyano, fluoro, chloro, méthoxy ou éthoxy,
R² représente le fluor, le chlore, le brome ou un reste méthyle, éthyle, n-propyle, isopropyle, méthoxy, éthoxy, n-propoxy, isopropoxy, méthylthio, éthylthio, n-propylthio, isopropylthio, méthylamino, éthylamino, n-propylamino, isopropylamino, diméthylamino ou diéthylamino, chacun éventuellement substitué par un radical cyano, fluoro, chlore, méthoxy ou éthoxy,
R³ représente l'hydrogène ou un reste éthyle ou méthyle éventuellement substitué par un radical méthoxy, éthoxy, n-propoxy, isopropoxy, acétyle, propionyle, n-butyroyle, isobutyroyle, méthoxycarbonyle, éthoxycarbonyle, n-propoxycarbonyle ou isopropoxycarbonyle,
R⁴ est un reste méthyle, éthyle, n-propyle, isopropyle, n-butyle, isobutyle ou sec.-butyle,
R⁵ est un reste méthyle, éthyle, n-propyle, isopropyle, n-butyle, isobutyle ou sec.-butyle et
R⁶ représente l'hydrogène, un groupe cyano, le fluor, le chlore, le brome, un reste méthyle, éthyle, n-propyle, isopropyle, n-butyle, isobutyle ou sec.-butyle, chacun éventuellement substitué par un radical cyano, fluoro, chloro, méthoxy ou éthoxy, un reste propényle, butényle, propynyle, ou butynyle, chacun éventuellement substitué par u radical cyano, fluoro ou chloro, un reste méthoxy, éthoxy, n-propoxy, isopropoxy, n-butoxy, isobutoxy, sec.-butoxy ou tertiobutoxy, chacun éventuellement substitué par un radical cyano, fluoro; chloro, méthoxy ou éthoxy, ou un reste propényloxy, butényloxy, propynyloxy ou butinyloxy, chacun éventuellement substitué par un radical cyano, fluoro ou chloro.

3. Composés de formule (I) suivant la revendication 1, **caractérisés en ce que** dans la formule
A représente l'azote ou un groupement CH,
E est une liaison simple,
Q représente l'oxygène ou le soufre,
R¹ représente l'hydrogène, le fluor, le chlore, le brome ou un groupe méthyle, éthyle, n-propyle, isopropyle, méthoxy, éthoxy, n-propoxy, isopropoxy, méthylthio, éthylthio, n-propylthio, isopropylthio, méthylamino, éthylamino, n-propylamino, isopropylamino, diméthylamino ou diéthylamino, chacun éventuellement substitué par un radical cyano, fluoro, chloro, méthoxy ou éthoxy,
R² représente le fluor, le chlore, le brome ou un reste méthyle, éthyle, n-propyle, isopropyle, méthoxy, éthoxy, n-propoxy, isopropoxy, méthylthio, éthylthio, n-propylthio, isopropylthio, méthylamino, éthylamino, n-propylamino, isopropylamino, diméthylamino ou diéthylamino, chacun éventuellement substitué par un radical cyano, fluoro, chloro, méthoxy ou éthoxy,
R³ représente l'hydrogène ou un reste éthyle ou méthyle éventuellement substitué par un radical méthoxy, éthoxy, n-propoxy, isopropoxy, acétyle, propionyle, n-butyroyle, isobutyroyle, méthoxycarbonyle, éthoxycarbonyle, n-propoxycarbonyle ou isopropoxycabonyle,
R⁴ est un reste méthyle, éthyle, n-propyle, isopropyle, n-butyle, isobutyle ou sec.-butyle,
R⁵ est un reste méthyle, éthyle, n-propyle, isopropyle, n-butyle, isobutyle ou sec.-butyle et
R⁶ représente l'hydrogène, un groupe cyano, le fluor, le chlore, le brome, un reste méthyle, éthyle, n-propyle, isopropyle, n-butyle, isobutyle ou sec.-butyle, chacun éventuellement substitué par un radical cyano, fluoro, chloro, méthoxy ou éthoxy, un reste propényle, butényle, propynyle ou butynyle, chacun éventuellement substitué par un radical cyano, fluoro ou chloro, un reste méthoxy, éthoxy, n-propoxy, isopropoxy, n- butoxy, isobutoxy, séc.-butoxy ou tertiobutoxy, chacun éventuellement substitué par un radical cyano, fluoro, chloro, méthoxy ou éthoxy, ou un reste propényloxy, butényloxy, propynyloxy ou butynyloxy, chaucn éventuellement substitué par un radical cyano, fluoro ou chloro.

4. Composés de formule (I) suivant la reve 1, **caractérisés en ce que** dans la formule
A représente l'azote ou un groupement CH,
E est un groupement NH,
Q représente l'oxygène ou le soufre,
R¹ représente l'hydrogène, le fluor, le chlore, le brome ou un reste méthyle, éthyle, n-propyle, isopropyle, méthoxy, éthoxy, n-propoxy, isopropoxy, méthylthio, éthylthio, n-propylthio, isopropylthio, méthylamino, éthylamino, n-propylamino, isopropylamino, diméthylamino ou diéthylamino, chacun éventuellement substitué par un radical cyano, fluoro, chloro, méthoxy ou éthoxy,
R² représente le fluor, le chlore, le brome ou un reste méthyle, éthyle, n-propyle, isopropyle, méthoxy, éthoxy, n-propoxy, isopropoxy, méthylthio, éthylthio, n-propylthio, isopropylthio, méthylamino, éthylamino, n-propylamino, isopropylamino, diméthylamino ou diéthylamino, chacun éventuellement substitué par un radical cyano, fluoro, chloro, méthoxy ou éthoxy,
R³ représente l'hydrogène ou un reste éthyle ou méthyle éventuellement substitué par un radical méthoxy, éthoxy, n-propoxy, isopropoxy, acétyle, propionyle, n-butyroyle, isobutyroyle, méthoxycarbonyle, éthoxycarbonyle, n-propoxycarbonyle ou isopropoxycarbonyle,
R⁴ est un reste méthyle, éthyle, n-propyle, isopropyle, n-butyle, isobutyle ou sec.-butyle,
R⁵ est un reste méthyle, éthyle, n-propyle, isopropyle, n-butyle, isobutyle ou sec.-butyle,
R⁶ représente l'hydrogène, un groupe cyano, le fluor, le chlore, le brome, un reste méthyle, éthyle, n-propyle, isopropyle, n-butyle, isobutyle ou sec.-butyle, chacun éventuellement substitué par un radical cyano, fluoro, chloro, méthoxy ou éthoxy, un reste propényle, butényle, propynyle ou butynyle, chacun éventuellement substitué par un radical cyano, fluoro ou chloro, un reste méthoxy, éthoxy, n-propoxy, isopropoxy, n- butoxy, isobutoxy, sec.-butoxy ou tertiobutoxy, chacun éventuellement substitué par un radical cyano, fluoro, chlore, méthoxy ou éthoxy, ou un reste propényloxy, butényloxy, propynyloxy ou butynyloxy, chacun éventuellement substitué par un radical cyano, fluoro ou chloro.

5. Procédé de production de composés de formule (I) suivant la revendication 1, **caractérisé en ce que**
- au cas où dans la formule générale (I), E représente une liaison simple -
(a) on fait réagir des aminoazines de formule générale (II) 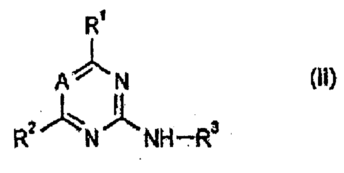 dans laquelle
A, R¹, R² et R³ ont la définition indiquée dans la revendication 1,
avec des iso(thio)cyanates de thiénylsulfonyle de formule générale (III) 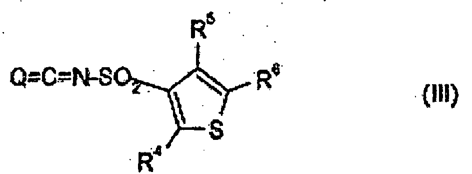 dans laquelle
Q, R⁴, R⁵ et R⁶ ont la définition indiquée dans la revendication 1,
éventuellement en présence d'un auxiliaire de réaction et, le cas échéant, en présence d'un diluant, ou bien
(b) on fait réagir des aminoazines substituées de formule générale (IV) 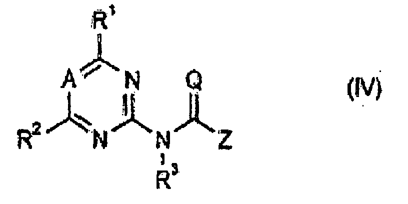 dans laquelle
A, Q, R¹, R² et R³ ont la définition indiquée dans la revendication 1 et
Z est un halogène, un reste alkoxy ou aryloxy,
avec des thiophènesulfonamides de formule générale (V) 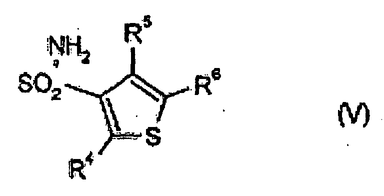 dans laquelle
R⁴, R⁵ et R⁶ ont la définition indiquée ci-dessus,
éventuellement en présence d'un auxiliaire de réaction et, le cas échant, en présence d'un diluant, ou bien
(c) on fait réagir des aminoazines de formule générale (II) 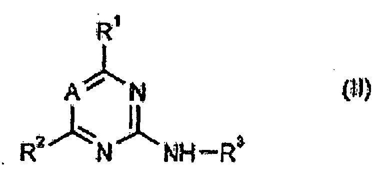 dans laquelle
A, R¹, R² et R³ ont la définition indiquée ci-dessus,
avec des (thio)amides thiophènesulfoniques substitués de formule générale (VI) 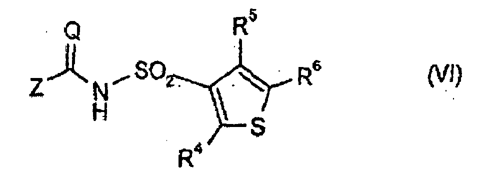 dans laquelle
Q, R⁴, R⁵ et R⁶ ont la définition indiquée ci-dessus et
Z est un halogène, un reste alkoxy ou aryloxy, éventuellement en présence d'un auxiliaire de réaction et, le cas échéant, en présence d'un diluant,
ou **en ce que** - au cas où E représente un groupement NH -
(d) on fait réagir des aminoazines de formule générale (II) 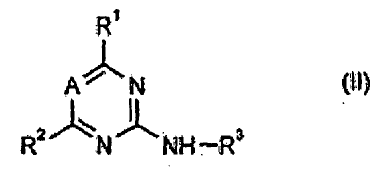 dans laquelle
A, R¹, R² et R³ ont la définition indiquée ci-dessus,
avec l'iso(thio)cyanate de chlorosulfonyle, éventuellement en présence d'un diluant, et on fait réagir les chlorosulfonylurées ainsi produites de formule générale (VII) 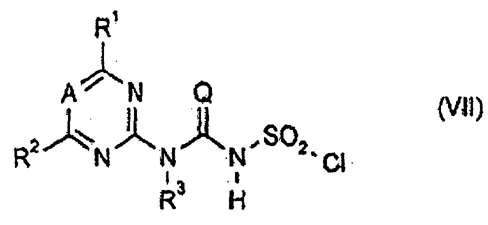 dans laquelle
A, Q, R¹, R² et R³ ont la définition indiquée ci-dessus, avec des aminothiophènes de formule générale (VIII) 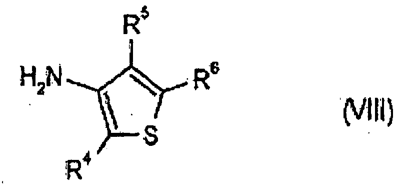
dans laquelle
R⁴, R⁵ et R⁶ ont la définition indiquée ci-dessus, éventuellement en présence d'un auxiliaire de réaction et, le cas échéant, en présence d'un diluant,
et on transforme éventuellement en sels par des modes opératoires usuels les composés de formule (I) obtenus selon les procédés (a), (b), (c) ou (d).

6. Composition herbicide, **caractérisée par** une teneur en au moins un composé de formule (I) ou en l'un de ses sels suivant la revendication 1.

7. Utilisation de composés de formule générale (I) ou de leurs sels, suivant la revendication 1, pour combattre une végétation indésirable.

8. Procédé pour combattre des mauvaises herbes, **caractérisé en ce qu'**on fait agir des composés de formule générale (I) ou leurs sels suivant la revendication 1 sur les mauvaises herbes ou sur leur milieu.

9. Procédé de préparation de compositions herbicides, **caractérisé en ce qu'**on mélange des composés de formule générale (I) ou leurs sels suivant la revendication 1 avec des diluants et/ou des agents tensioactifs.
